Europäisches Patentamt
European Patent Office
Office européen des brevets

(19)

(11) Publication number: **0 446 604 A2**

(12) **EUROPEAN PATENT APPLICATION**

(21) Application number: **91101496.7**

(22) Date of filing: **05.02.91**

(51) Int. Cl.[5]: **C07D 401/06**, C07D 471/04, C07D 401/14, C07D 213/85, A61K 31/435, C07D 495/04

(30) Priority: **16.03.90 US 494387**

(43) Date of publication of application: **18.09.91 Bulletin 91/38**

(84) Designated Contracting States: **AT BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Tomcufcik, Andrew Stephen**
**48 Dearborn Drive**
**Old Tappan, New Jersey 07675(US)**
Inventor: **Meyer, Walter Edward**
**40 Van Orden Avenue**
**Suffern, New York 10901(US)**
Inventor: **Marsico, Joseph William**
**106 S. Middletown Road**
**Pearl River, New York 10965(US)**

(74) Representative: **Wächtershäuser, Günter, Dr.**
**Tal 29**
**W-8000 München 2(DE)**

(54) **Pyridine and related aza heterocycle derivatives as cardiovascular agents.**

(57) Novel pharmaceutical compounds and compositions having nitrogen containing ring systems which may be represented by the following structural formula:

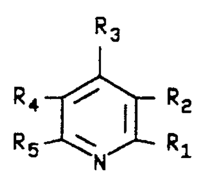

wherein $R_1$ or $R_3$ is a moiety of the formula:

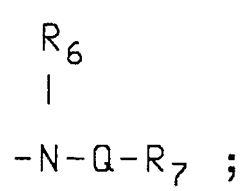

wherein $R_6$ is selected from either hydrogen or acetyl; $R_7$ is selected from 2, 3 or 4-pyridyl or 1-imidazolyl and Q is $-(CH_2)_n$, where n is an integer from 1 to 5 and $R_1$ and $R_2$, $R_2$ and $R_3$, $R_3$ and $R_4$ or $R_4$ and $R_5$ taken together may be $-CH=CH-CH=CH-$. The compounds and compositions are useful as inhibitors of thromboxane synthetase and in the treatment of hypertension and arrythmia in mammals.

EP 0 446 604 A2

This invention relates to new organic compounds and, more particularly, is concerned with novel nitrogen containing ring systems which may be represented by the following structural formula:

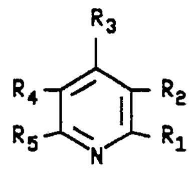

therein $R_1$ is selected from the group consisting of hydrogen, halogen or

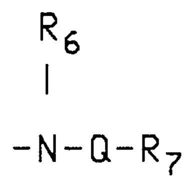 ;

$R_2$ is selected from the group consisting of hydrogen, nitro, nitrile or $R_1$ and $R_2$ taken together may be -CH=CH-CH=CH-; $R_3$ is selected from the group consisting of hydrogen, alkyl $C_1$-$C_3$, phenyl,

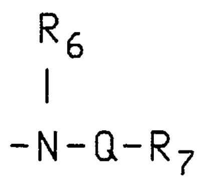

or $R_2$ and $R_3$ taken together may be -CH=CH-CH=CH-; $R_4$ is selected from the group consisting of hydrogen, nitro, nitrile, alkyl $C_1$-$C_3$,

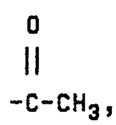, or 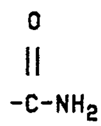 ;

or $R_3$ and $R_4$ taken together may be:

-CH=CH-CH=CH- ;

$R_5$ is selected from the group consisting of hydrogen, alkyl $C_1$-$C_3$, halogen, phenyl, 3-(trifluoromethyl)-phenyl, 3-fluorophenyl, 3,4,5-trimethoxyphenyl, and 2,3 or 4-pyridyl or $R_4$ and $R_5$ taken together may be selected from the following formulae:

$-CH=CH-CH=CH-$ ,

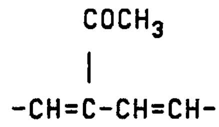 , 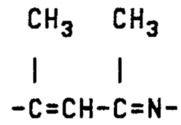 ,

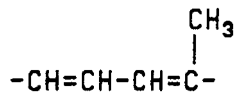 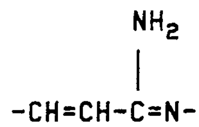 ,

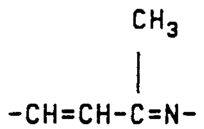 , 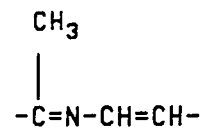 ,

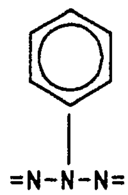 ,

$-S-CH=CH-$ ;

$R_6$ is selected from either hydrogen or acetyl; $R_7$ is selected from either 2,3, or 4-pyridyl or 1-imidazolyl; Q is selected from the group consisting of $-(CH_2)_n$,where n is an integer from 1 to 5, straight or branched chain alkyl; with the proviso that in all instances, either $R_1$ or $R_3$ must be the substituent:

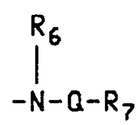

as defined above, together with the pharmacologically acceptable salts thereof.

As used herein, the term "halogen" denotes all halogens, that is, bromine, chlorine, fluorine and iodine.

The organic bases of this invention form non-toxic acid-addition salts with a variety of pharmacologically acceptable organic and inorganic salt-forming reagents. Thus acid-addition salts, formed by admixture of the organic free base with one or more equivalents of an acid, suitably in a neutral solvent, are formed with such acids as sulfuric, phosphoric, hydrochloric, hydrobromic, hydriodic, sulfamic, citric, lactic, malic, succinic, maleic, fumaric, tartaric, acetic, benzoic, gluconic, ascorbic and the like. For the purpose of this invention the free bases are equivalent to their non-toxic acid-addition salts.

Preferred are those compounds having a quinoline moiety of the formula:

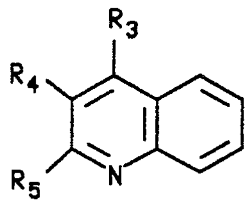

or

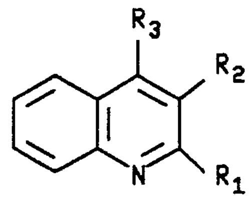

wherein $R_1$ is selected from the group consisting of hydrogen, halogen, or

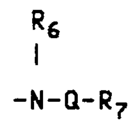 ;

$R_2$ is selected from the group consisting of hydrogen, nitro, or nitrile; $R_3$ is selected from the group consisting of hydrogen, alkyl $C_1$-$C_3$, phenyl, or

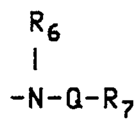

$R_4$ is selected from the group consisting of hydrogen, nitro, nitrile, alkyl $C_1$-$C_3$,

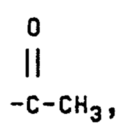 , or 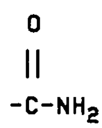 ;

$R_5$ is selected from the group consisting of hydrogen, alkyl $C_1$-$C_3$, halogen, phenyl, 3-(trifluoromethyl)-phenyl, 3-fluorophenyl, 3,4,5-trimethoxyphenyl, and 2,3 or 4-pyridyl; $R_6$ is selected from either hydrogen or acetyl; $R_7$ is selected from 2,3 or 4-pyridyl or 1-imidazolyl; Q is selected from the group consisting of $-(CH_2)_n$ straight or branched chain alkyl where n is an integer from 1 to 5; with the proviso that, in all instances, either $R_1$ or $R_3$ must be:

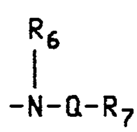

as hereinbefore defined; and the pharmacologically acceptable salts thereof.

Also preferred are those compounds of the formulae:

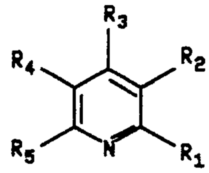

wherein $R_1$ is selected from the group consisting of hydrogen, halogen or

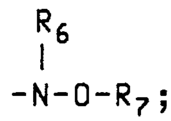

$R_2$ is selected from the group consisting of hydrogen, nitro or nitrile;
$R_3$ is selected from the group consisting of hydrogen, alkyl $C_1$-$C_3$, phenyl or

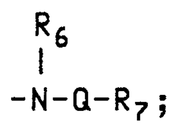

and $R_4$ and $R_5$ taken together is selected from the following formulae:

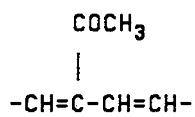 , 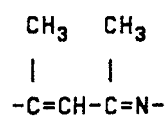 ,

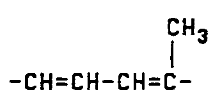 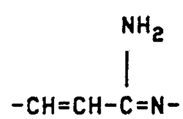 ,

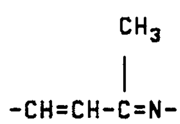 , 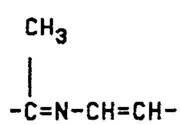 ,

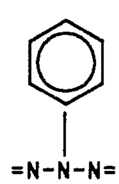 ,

-S-CH=CH- ;

$R_6$ is selected from either hydrogen or acetyl; $R_7$ is selected from either 2,3, or 4-pyridyl or 1-imidazolyl; Q

is selected from the group consisting of $-(CH_2)_n$, where n is an integer from 1 to 5, straight or branched chain alkyl; with the proviso that in all instances, either $R_1$ or $R_3$ must be the substituent:

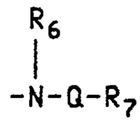

as hereinbefore defined; and the pharmacologically acceptable salts thereof.

Detailed Description of the Invention

The compounds of the present invention may be readily prepared according to the following reaction scheme, wherein $R_1$, $R_2$, $R_3$, $R_4$, $R_5$, $R_6$, $R_7$ and Q are as described herein above.

The 2-substituted pyridine and quinoline compounds of the present invention may be prepared according to the following Scheme I.

Scheme I

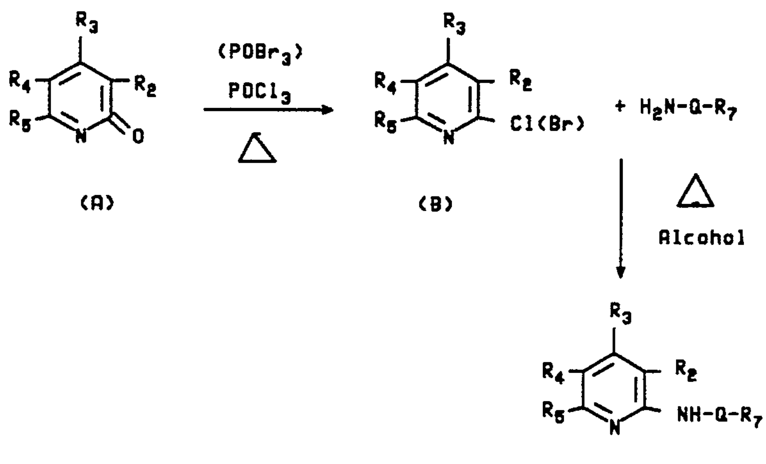


(A) (B) (C)

In accordance with the above reaction scheme a substituted halo nitrogen-containing ring system (B) is reacted with an appropriate $R_7$-Q-amine (i.e. 1H-imidazole-1-propanamine, 1H-imidazole-1-butanamine etc.) in a suitable solvent such as ethanol at reflux temperature for several hours giving the desired compounds. Compound C may be further reacted with sodium hydride and an appropriate anhydride to give compounds of type D as set forth below in Scheme II.

Scheme II

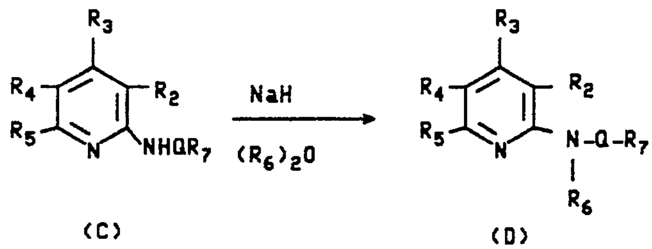


(C) (D)

The 4-substituted pyridine and quinoline compounds are prepared according to Scheme III below wherein a substituted halo nitrogen-containing ring system (F) is reacted with the appropriate $R_7$-Q- amine in a suitable solvent such as ethanol at reflux temperature for several hours giving the desired compounds (G).

Scheme III:

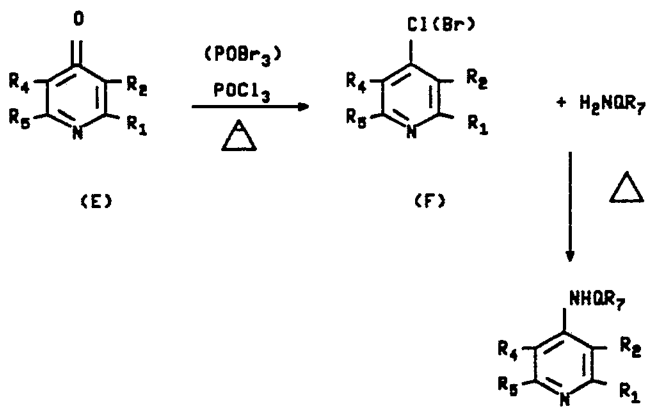


(E) (F) (G)

The isoquinoline compounds of the present invention are prepared according to the following procedure:

Scheme IV:

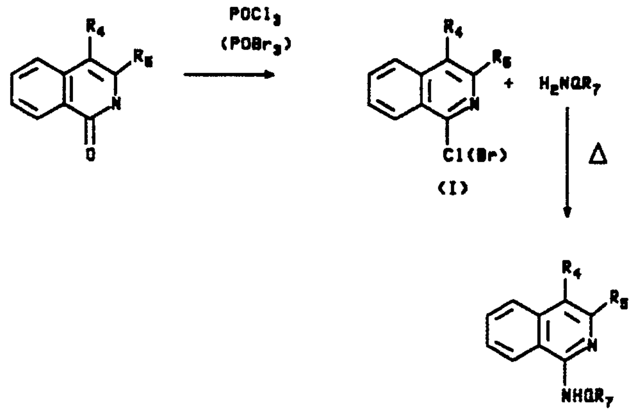

(I)

(J)

In accordance with the foregoing reaction Scheme IV, a halo substituted isoquinoline ring system (I) is reacted with the appropriate $R_7$-Q-amine in a suitable solvent such as phenol at reflux temperature for several hours giving the desired compounds (J).

Inhibition of Thromboxane Synthetase

The compounds of this invention inhibit thromboxane synthetase enzyme. Thus, these compounds are useful in the treatment of diseases characterized by an imbalance of thromboxane $A_2$/prostacyclin such as ischemic heart disease, transient ischemic attack, thrombosis and migraine. Recent reviews have established the role of the thromboxane/prostacyclin balance in the vascular system [cardiovascular Diseases: New Trends in Surgical and Medical Aspects, H. Barnett, P. Paoletti, E. Flamm and G. Brambilla, eds., Elsevier/ North-Holland Biomedical Press, pp 137-150 (1981)]. Prostacyclin ($PGI_2$) is a potent vasodilator and platelet aggregation inhibitor, whereas thromboxane ($TXA_2$) is a powerful vasoconstrictor and causative of platelet aggregation. $TXA_2$ is synthesized by thromboxane synthetase enzyme located in, for example, blood platelets. When $TXA_2$ production is increased relative to $PGI_2$, platelet aggregation, thrombosis and vasospasm may occur [Lancet (i), 1216 (1977); Lancet, 479 (1977); Science, 1135 (1976); Amer. J. Cardiology, 41, 787 (1978)]. $TXA_2$ synthetase inhibitors have been shown to have superior anti-thrombotic action to that of aspirin [J. Clin. Invest., 65, 400 (1980); Br. J Pharmac., 76, 3 (1982)].

The role of prostaglandins including $TXA_2$ and $PGI_2$ in ischemic heart patients has been reviewed [Cardiovascular Pharmacology of the Prostaglandins, A. G. Herman, P. M. Vanhoute, H. Denolin and A. Goosens, eds., Raven Press, New York, pp 361-374 (1982)]. Injection of $TXA_2$ into coronary arteries of guinea pigs and rabbits causes myocardial ischemia and subendocardial necrosis [Drugs of the Future, 7, 331 (1982); Proc. Jap. Acad., 53(B), 38 (1977); Eur. J. Pharmacol., 53, 49 (1978)]. Recent research has demonstrated the beneficial effects of $PGI_2$ and selective inhibition of thromboxane synthetase on ischemic myocardium in canines [J. Cardiovascular Pharmacology, 4, 129 (1982)]. Thus compounds which selectively inhibit thromboxane synthetase (and hence $TXA_2$) without adversely affecting $PGI_2$ are useful in the treatment of vascular diseases such as ischemia and migraine. In addition, inhibition of $TXA_2$ formation may effectively treat platelet aggregation and prevent thrombosis.

Under urethan anesthesia, 10 $\mu$1 of arterial blood from Okamoto-Aoki spontaneously hypertensive rats (SHR) (Taconic Farms, Germantown, NY) between 19 and 24 weeks in age is collected in one ml of 3.2% sodium citrate in a polystyrene tube. The blood is diluted with 3 ml of cold saline and centrifuged at room temperature for 15 minutes at 460xg. The platelet rich plasma (PRP) is separated. The platelets are isolated by centrifuging the PRP for 10 minutes at 1060xg and are washed in 4 ml of cold oxygenated Krebs phosphate buffer, pH 7.4. The chilled platelets, recovered from centrifuging at 800xg for 10 minutes, are resuspended in oxygenated Krebs phosphate buffer and diluted to contain 4.5-6.0 x $10^4$ platelets/$\mu$1.

The inhibition of thromboxane (TX) formation is studied by determining the concentration of thromboxane $B_2$ ($TXB_2$), the stable hydrolysis product of $TXA_2$. Assay samples, prepared on ice, contain 200 $\mu$1 platelet suspension, 50 $\mu$1 saline, and 50 $\mu$1 vehicle (saline) or drug under study.

The samples are incubated for 10 minutes at 37°C in a metabolic shaker. The reaction is terminated by immersing the tubes in an ice bath and adding 50 $\mu$1 of 0.5M citric acid. The samples are centrifuged for 10 minutes in a refrigerated centrifuge and the supernatants thus obtained are decanted and stored at -20°C.

The $TXB_2$ content for each sample is determined by a direct radioimmunoassay (RIA) utilizing a $TXB_2$ specific RIA kit purchased from New England Nuclear, Boston, MA and expressed as pg $TXB_2$ formed per minute per sample, from which the percent inhibition of $TXB_2$ formation is calculated.

Table I shows the percentage of thromboxane synthetase enzyme inhibition when representative compounds of this invention are employed in the assay described above.

## Table I

### Thromboxane Synthetase Enzyme Inhibition

| Compound | Example # | Concentration | % Inhibition |
|---|---|---|---|
| N-[3-(1H-Imidazol-1-yl)propyl]-3-nitro-2-pyridinamine | 2 | $1x10^{-4}M$ | 80 |
| 2-[[3-(1H-Imidazol-1-yl)propyl]amino]-6-methyl-3-pyridinecarbonitrile | 3 | $1x10^{-4}M$ | 79 |
| 2-[[3-(1H-Imidazol-1-yl)propyl]amino]-3-pyridinecarbonitrile | 4 | $1x10^{-4}M$ | 93 |
| N-[3-(1H-Imidazol-1-yl)propyl]-2-quinolinamine | 5 | $1x10^{-4}M$ | 93 |

**Table I (Cont'd)**

**Thromboxane Synthetase Enzyme Inhibition**

| Compound | Example # | Concentration | % inhibition |
|---|---|---|---|
| N-[3-(1H-imidazol-1-yl) propyl]-4-methyl-2-quinolinamine | 6 | $1x10^{-4}M$ | 98 |
| N-[3-(1H-imidazol-1-yl) propyl]-6-phenanthridinamine | 8 | $1x10^{-4}M$ | 100 |
| N-(3-Nitro-2-pyridinyl)-4-pyridinebutanamine,(E)-2-butenedioate (1:1) | 100 | $1x10^{-4}M$ | 88 |
| N-[3-(1H-Imidazol-1-yl)-2-methylpropyl]-6-phenanthridinamine | 86 | $1x10^{-4}M$ | 100 |
| N-[3-(1H-Imidazol-1-yl)-2-methylpropyl]-4-methyl-2-quinolinamine | 88 | $1x10^{-4}M$ | 98 |
| N-[3-(1H-Imidazol-1-yl) butyl]-6-phenanthridinamine | 89 | $1x10^{-4}M$ | 98 |
| 5-Acetyl-2-[[3-(1H-imidazol-1-yl)propyl]amino]-6-methyl-3-pyridinecarbonitrile | 12 | $1x10^{-4}M$ | 96 |
| N-[3-(1H-Imidazol-1-yl) propyl]-1-isoquinolinamine | 14 | $1x10^{-4}M$ | 84 |

**Table I (Cont'd)**

**Thromboxane Synthetase Enzyme Inhibition**

| Compound | Example # | Concentration | % Inhibition |
|---|---|---|---|
| 2-[[3-(1*H*-Imidazol-1-yl)propyl]amino]-6-[(3-trifluoromethyl)phenyl]-3-pyridinecarbonitrile | 18 | $1x10^{-4}M$ | 98 |
| *N*-[4-(3-Pyridinyl)butyl]-1-isoquinolinamine | 105 | $1x10^{-4}M$ | 97 |
| *N*-[3-(1*H*-Imidazol-1-yl)propyl]-6-methoxy-4-methyl-2-quinolinamine | 21 | $1x10^{-4}M$ | 97 |
| 6-Methoxy-4-methyl-*N*-[4-(3-pyridinyl)butyl]-2-quinolinamine | 106 | $1x10^{-4}M$ | 97 |
| *N*-[3-(1*H*-Imidazol-1-yl)propyl]-5,7-dimethyl-1,8-naphthyridin-2-amine | 24 | $1x10^{-4}M$ | 93 |
| *N*-[3-(1*H*-Imidazol-1-yl)propyl]-1,8-naphthyridin-2-amine,(E)-2-butenedioate(1:2) | 27 | $1x10^{-4}M$ | 90 |
| 6-[[3-(1*H*-Imidazol-1-yl)propyl]amino]-2-methyl-3-pyridinecarbonitrile | 30 | $1x10^{-4}M$ | 94 |

**Table I (Cont'd)**

**Thromboxane Synthetase Enzyme Inhibition**

| Compound | Example # | Concentration | % Inhibition |
|---|---|---|---|
| N-[3-(1H-Imidazol-1-yl) propyl]-4,8-dimethyl-2-quinolinamine | 32 | $1x10^{-4}M$ | 98 |
| 4,8-Dimethyl-N-[4-(3-pyridinyl)butyl]-2-quinolinamine | 107 | $1x10^{-4}M$ | 99 |
| $N^2$-[3-(1H-Imidazol-1-yl) propyl]-4-methyl-1,8-naphthyridin-2,7-diamine | 34 | $1x10^{-4}M$ | 98 |
| N-[3-(1H-Imidazol-1-yl) propyl]-7-methyl-1,8-naphthyridin-2-amine,(E)-2-butenedioate(1:2) | 38 | $1x10^{-4}M$ | 98 |
| 6-Methyl-2-[[4-(3-pyridinyl)butyl]amino]-3-pyridinecarbonitrile | 108 | $1x10^{-4}M$ | 96 |
| N-[3-(1H-Imidazol-1-yl) propyl]-5-methyl-1,6-naphthyridin-2-amine,(E)-2-butenedioate(2:3) | 40 | $1x10^{-4}M$ | 95 |
| 5,7-Dimethyl-N-[4-(3-pyridinyl)butyl]-1,8-naphthyridin-2-amine,(E)-2-butenedioate (1:2) | 109 | $1x10^{-4}M$ | 97 |

Table I (Cont'd)

Thromboxane Synthetase Enzyme Inhibition

| Compound | Example # | Concentration | % Inhibition |
|---|---|---|---|
| N-[3-(1H-Imidazol-1-yl) propyl]-4-phenyl-2-quinolinamine | 44 | $1x10^{-4}M$ | 100 |
| 4-Phenyl-N-[4-(3-pyridinyl) butyl]-2-quinolinamine | 110 | $1x10^{-4}M$ | 99 |
| 6-[[3-(1H-Imidazol-1-yl) propyl]amino]-3-pyridinecarbonitrile | 45 | $1x10^{-4}M$ | 92 |
| 6-[[3-(1H-Imidazol-1-yl) propyl]amino]-3-pyridinecarboxamide | 46 | $1x10^{-4}M$ | 80 |
| 6-Chloro-N-[3-(1H-imidazol-1-yl)propyl]-2-pyridinamine | 47 | $1x10^{-4}M$ | 91 |
| N-[3-(1H-Imidazol-1-yl) propyl]-8-methyl-4-phenyl-2-quinolinamine | 51 | $1x10^{-4}M$ | 89 |
| 2-[[3-(1H-Imidazol-1-yl) propyl]amino]-6-(4-pyridinyl)-3-pyridine-carbonitrile | 52 | $1x10^{-4}M$ | 85 |

**Table I (Cont'd)**

**Thromboxane Synthetase Enzyme Inhibition**

| Compound | Example # | Concentration | % Inhibition |
|---|---|---|---|
| 8-Methyl-4-phenyl-N-[4-(3-pyridinyl)butyl]-2-quinolinamine | 111 | $1x10^{-4}M$ | 85 |
| N-[3-(1H-Imidazol-1-yl)propyl]-2-phenyl-2H-1,2,3-triazolo[4,5-b]pyridin-5-amine | 56 | $1x10^{-4}M$ | 95 |
| 6-Chloro-N-[3-(1H-imidazol-1-yl)propyl]-3-nitro-2-pyridinamine, monohydrochloride | 57 | $1x10^{-4}M$ | 94 |
| N-[3-(1H-Imidazol-1-yl)propyl]-N-(5-nitro-2-pyridinyl)acetamide | 58 | $1x10^{-4}M$ | 83 |
| N-[3-(1H-Imidazol-1-yl)propyl]thieno[3,2-b]pyridin-5-amine | 62 | $1x10^{-4}M$ | 91 |
| 6-[[3-(1H-imidazol-1-yl)propyl]amino]-2-[3-(trifluoromethyl)phenyl]-3-pyridinecarbonitrile,(E)-2-butenedioate (1:1) | 71 | $1x10^{-4}M$ | 100 |

**Table I (Cont'd)**

**Thromboxane Synthetase Enzyme Inhibition**

| Compound | Example # | Concentration | % Inhibition |
|---|---|---|---|
| 6-Methyl-2-[(3-pyridinylmethyl)amino]-3-pyridinecarbonitrile | 91 | $1x10^{-4}M$ | 81 |
| 6-Methyl-2-[(4-pyridinylmethyl)amino]-3-pyridinecarbonitrile | 93 | $1x10^{-4}M$ | 86 |
| N-[3-(1H-Imidazol-1-yl)propyl]-N-(4-Methyl-2-quinolinyl)acetamide, monoacetate | 83 | $1x10^{-4}M$ | 90 |

The novel compounds of the present invention are found to be highly useful for inhibiting thromboxane synthetase in mammals when administered in amounts ranging from about 1.0 mg to about 20.0 mg/kg of body weight per day. A preferred dosage regimen for optimum results would be from about 1.0 mg to about 10.0 mg/kg of body weight per day. Such dosage units are employed that a total of from about 70 to about 700 mg of active compound for a subject of about 70 kg of body weight are administered in a 24 hour period. This dosage regimen may be adjusted to provide the optimum therapeutic response.

For example, several divided doses may be administered daily or the dose may be proportionally reduced as indicated by the exigencies of the therapeutic situation. The compounds of this invention are preferably administered orally but may be administered in any convenient manner such as by the intravenous, intramuscular, or subcutaneous routes.

Hypotensive Activity in Spontaneously Hypertensive Rats

The novel compounds of the present invention are active hypotensive agents and were tested for hypotensive activity by the method of P.S. Chan and D. Poorvin, Clinical and Experimental Hypertension, 1 (6), 817-830 (1979). Male, 16 week old, spontaneously hypertensive rats of the Okamoto strain, from Taconic Farms, Germantown, New York, having an average mean arterial blood pressure of 160±1.5 mm of mercury, were used in the test. One to four rats were used per test compound. A rat is dosed by gavage with a test compound suspended in 2% pre-boiled starch at a concentration of 50 mg/ml, at a dose of 100 mg/kg of body weight or less, with 0.9% sodium chloride loading at a dose of 25 ml/kg of body weight. A second identical dose of the test compound, without sodium chloride loading is given 24 hours later. At 28 hours after the initial dose the mean arterial blood pressure (MABP) is measured. The procedure is repeated in a second and third rat when necessary.

The results of this test on representative compounds of the present invention appear below in Table II.

**Table II**
**Hypotensive Activity**

| Compound | Example# | Avg. MABP in mm Hg (No. of Rats) |
|---|---|---|
| N-[3-(1H-Imidazol-1-yl) propyl]-5-nitro-2-pyridinamine,(Z)-2-butenedioate (1:1) | 1 | 121 (4) |
| N-[3-(1H-Imidazol-1-yl) propyl]-3-nitro-2-pyridinamine | 2 | 98 (2) |
| 2-[[3-(1H-Imidazol-1-yl) propyl]amino]-6-methyl-3-pyridinecarbonitrile | 3 | 80 (2) |
| 2-[[3-(1H-Imidazol-1-yl)propyl] amino]-3-pyridinecarbonitrile | 4 | 117 (4) |
| N-[3-(1H-Imidazol-1-yl) propyl]-2-quinolinamine | 5 | 97 (2) |
| N-[3-(1H-Imidazol-1-yl) propyl]-4-methyl-2-quinolinamine | 6 | 100 (3) |
| N-[3-(1H-Imidazol-1-yl) propyl]-6-phenanthridinamine | 8 | 103 (2) |

Table II (Cont'd)
Hypotensive Activity

| Compound | Example# | Avg. MABP in mm Hg (No. of Rats) |
|---|---|---|
| N-[4-(1H-Imidazol-1-yl) butyl]-4-methyl-2-quinolinamine | 101 | 96 (2) |
| N-[3-(1H-Imidazol-1-yl)-2-methylpropyl]-6-phenanthridinamine | 86 | 114 (2) |
| 2-[[5-(1H-Imidazol-1-yl) pentyl]amino]-6-methyl-3-pyridinecarbonitrile | 90 | 116 (2) |
| N-[3-(1H-Imidazol-1-yl) butyl]-4-methyl-2-quinolinamine | 87 | 111 (2) |
| 4-Methyl-N-4-(3-pyridinyl-butyl)-2-quinolinamine, dihydrochloride | 103 | 87 (2) |
| N-[3-(1H-Imidazol-1-yl) 2-methylpropyl]-4-methyl-2-quinolinamine | 88 | 96 (2) |
| N-[3-(1H-Imidazol-1-yl) propyl]-5-nitro-2-pyridinamine | 11 | 119 (2) |

**Table II (Cont'd)**

**Hypotensive Activity**

| Compound | Example# | Avg. MABP in mm Hg (No. of Rats) |
|---|---|---|
| N-[4-(1H-Imidazol-1-yl) butyl]-6-phenanthridinamine | 104 | 93 (1) |
| N-[3-(1H-Imidazol-1-yl) propyl]-1-isoquinolinamine | 14 | 121 (3) |
| N-[3-(1H-Imidazol-1-yl) propyl]-6-methoxy-4-methyl-2-quinolinamine | 21 | 110 (2) |
| 6-Methoxy-4-methyl-N-[4-(3-pyridinyl)butyl]-2-quinolinamine | 106 | 120 (2) |
| 6-[[3-(1H-Imidazol-1-yl) propyl]amino]-2-methyl-3-pyridinecarbonitrile | 30 | 61 (2) |
| N-[3-(1H-Imidazol-1-yl) propyl]-4,8-dimethyl-2-quinolinamine | 32 | 96 (2) |

**Table II (Cont'd)**
**Hypotensive Activity**

| Compound | Example# | Avg. MABP in mm Hg (No. of Rats) |
|---|---|---|
| N-[3-(1H-Imidazol-1-yl) propyl]thieno[3,2-b]-pyridin-5-amine | 62 | 105 (2) |
| 2-[[3-(1H-Imidazol-1-yl) propyl]amino]-3-quinoline-carbonitrile | 82 | 94 (2) |
| 4-Methyl-N-[3-(3-pyridinyl) propyl]-2-quinolinamine, dihydrochloride | 98 | 95 (2) |

The compounds of this invention are also active anti-arrythmic agents as established in the following test.

Calcium (or Potassium)-Chloride-Induced Arrythmia in Mice

Intravenous administration of high doses of calcium or potassium chloride in animals produces cardiac arrhythmias, cardiac fibrillation and cardiac arrest. Any agent that can prevent these events is considered an antiarrhythmic/antifibrillatory agent. (Meth. and Find Exptl. Clin Pharmacol.2:223-252(1980); Pharmacol, Ther. 24:401-433 (1984); Experimental Cardiac and Antiarrhythmic Drugs, L. Szekeres and Gy. J. Dapp, Akademiai Kiado Preso, Budapest, 9-448 (1971).)

The method employed is as follows: Male mice of the Swiss Webster Strain (Charles River, Wilmington, MA) weighing 25-35 g are anesthetized with ethyl carbamate (urethan) (1.5g/kg body weight i.p.). A 25-gauge needle attached to PE-20 tubing is inserted into a caudal vein for drug administration. The lead II ECG is recorded by needle electrodes placed subcutaneously in the limbs. All data is collected on a Gould Brush recorder. Animals are observed 5 minutes before drug administration. A 5, 15, or 30 minute period following drug administration is allowed before the bolus injection of calcium chloride, 200 mg/kg i.v. or potassium chloride, 62.5 mg/kg i.v. Control mice receive 4 ml/kg of 0.9% sodium chloride i.v. Mice are observed an additional 15 minutes for arrhythmias, fibrillation, survival or death. Any compound at a dose of less than 20 mg/kg i.v. that protects 3 out of 6 mice from cardiac death in either the potassium or calcium chloride test, is considered active.

The results of this test on representative compounds of the present invention appear in Table III.

## TABLE III

### Antiarrhythmic/Antifibrillatory Activity of Chemicals in Anesthetized Mice

| Compound Dose = 10 mg/kg i.v. | Example # | $CaCl_2$ #Mice Surviving / #Mice Tested | KCl #Mice Surviving / #Mice Tested |
|---|---|---|---|
| N-[3-(1H-Imidazol-1-yl)propyl]-3-nitro-2-pyridinamine | 2 | 3/6 | 4/6 |
| 2-[[3-(1H-Imidazol-1-yl)propyl]amino]-3-pyridinecarbonitrile | 4 | 4/6 | 1/6 |
| N-[3-(1H-Imidazol-1-yl)propyl]-2-quinolinamine | 5 | 5/6 | 6/12 |
| N-[3-(1H-Imidazol-1-yl)propyl]-6-phenanthridinamine | 8 | 2/6 | 6/6 |
| N-(3-Nitro-2-pyridinyl)-4-pyridinebutanamine | 100 | 0/6 | 6/6 |
| N-(3-Nitro-2-pyridinyl)-4-pyridinebutanamine, (E)-2-butenedioate (1:1) | 100 | 3/6 | 2/6 |
| 2-[[3-(1H-Imidazol-1-yl)-2-methylpropyl]amino]-6-methyl-3-pyridinecarbonitrile, dihydrochloride | 84 | 3/6 | 5/6 |
| 2-[[3-(1H-Imidazol-1-yl)butyl]amino]-6-methyl-3-pyridinecarbonitrile,dihydrochloride | 85 | 2/6 | 5/6 |

TABLE III (Cont'd)

Antiarrhythmic/Antifibrillatory Activity of Chemicals in Anesthetized Mice

| Compound Dose = 10 mg/kg i.v. | Example # | $CaCl_2$ #Mice Surviving / #Mice Tested | KCl #Mice Surviving / #Mice Tested |
|---|---|---|---|
| N-[3-(1H-Imidazol-1-yl)-2-methylpropyl]-6-phenanthridinamine | 86 | 1/6 | 3/6 |
| 2-[[5-(1H-Imidazol-1-yl)pentyl]amino]-6-methyl-3-pyridinecarbonitrile | 90 | 0/6 | 3/6 |
| 4-Methyl-N-[2-(3-pyridinyl)ethyl]-2-quinolinamine, dihydrochloride | 95 | 1/6 | 4/6 |
| N-[3-(1H-Imidazol-1-yl)-2-methylpropyl]-4-methyl-2-quinolinamine | 88 | 1/6 | 5/6 |
| N-[3-(1H-Imidazol-1-yl)propyl]-6-methyl-2-pyridinamine,(E)-2-butenedioate (2:3) | 10 | 0/6 | 3/6 |
| N-[3-(1H-Imidazol-1-yl)propyl]-5-nitro-2-pyridinamine | 11 | 0/6 | 3/6 |
| 4-Methyl-N-[3-(3-pyridinyl)propyl]-2-quinolinamine, dihydrochloride | 98 | 5/6 | 5/6 |
| 5-Acetyl-2[[3-(1H-Imidazol-1-yl)propyl]amino]-6-methyl-3-pyridinecarbonitrile | 12 | 1/6 | 4/6 |
| N-[4-(3-Pyridinyl)butyl]-1-isoquinolinamine, dihydrochloride | 105 | 3/6 | 4/6 |

TABLE III (Cont'd)

Antiarrhythmic/Antifibrillatory Activity of Chemicals in Anesthetized Mice

| Compound Dose = 10 mg/kg i.v. | Example # | $CaCl_2$ #Mice Surviving / #Mice Tested | KCl #Mice Surviving / #Mice Tested |
|---|---|---|---|
| N-[3-(1H-Imidazol-1-yl)propyl]-5,7-dimethyl-1,8-naphthyridin-2-amine | 24 | 1/6 | 6/6 |
| 6-[[3-(1H-Imidazol-1-yl)propyl]amino]-2-methyl-3-pyridinecarbonitrile | 30 | 1/6 | 6/6 |
| N-[3-(1H-Imidazol-1-yl)propyl]-4,8-dimethyl-2-quinolinamine | 32 | 0/6 | 3/6 |
| 4,8-Dimethyl-N-[4-(3-pyridinyl)butyl]-2-quinolinamine | 107 | 0/6 | 5/6 |
| $N^2$-[3-(1H-Imidazol-1-yl)propyl]-4-methyl-1,8-naphthyridine-2,7-diamine | 34 | 3/6 | 4/6 |
| N-[3-(1H-Imidazol-1-yl)propyl]-7-methyl-1,8-naphthyridin-2-amine,(E)-2-butenedioate(1:2) | 38 | 2/6 | 4/6 |
| 6-Methyl-2-[[4-(3-pyridinyl)butyl]amino]-3-pyridinecarbonitrile | 108 | 4/6 | 5/6 |
| N-[3-(1H-Imidazol-1-yl)propyl]-5-methyl-1,6-naphthyridin-2-amine,(E)-2-butenedioate(2:3) | 40 | 2/6 | 3/6 |
| 6-Methyl-2-[[2-(4-pyridinyl)ethyl]amino]-3-pyridinecarbonitrile, dihydrochloride | 97 | 0/6 | 3/6 |

Compositions according to the present invention having the desired clarity, stability and adaptability for parenteral use are obtained by dissolving from 0.10% to 10.0% by weight of active compound in a vehicle consisting of a polyhydric aliphatic alcohol or mixtures thereof. Especially satisfactory are glycerin, propylene glycol, and polyethylene glycols. The polyethylene glycols consist of a mixture of non-volatile, normally liquid, polyethylene glycols which are soluble in both water and organic liquids and which have a

molecular weight of from about 200 to 1500. Although the amount of active compound dissolved in the above vehicle may vary from 0.10% to 10.0% by weight, it is preferred that the amount of active compound employed be from about 3.0 to about 9.0% by weight. Although various mixtures of the aforementioned non-volatile polyethylene glycols may be employed, it is preferred to use a mixture having an average molecular weight of from about 200 to about 400.

In addition to the active compound, the parenteral solutions may also contain various preservatives which may be used to prevent bacterial and fungal contamination. The preservatives which may be used for these purposes are, for example, myristyl-gamma-picolinium chloride, benzalkonium chloride, phenethyl alcohol, p-chlorophenyl-glycerol ether, methyl and propyl parabens, and thimerosal. As a practical matter, it is also convenient to employ antioxidants. Suitable antioxidants include, for example, sodium bisulfite, sodium metabisulfite, and sodium formaldehyde sulfoxylate. Generally, from about 0.05 to about 0.2% concentrations of antioxidant are employed.

For intramuscular injection, the preferred concentration of active compound is 0.25 to 0.50 mg/ml of the finished compositions. The novel compounds of the present invention are equally adapted to intravenous administration when diluted with water or diluents employed in intravenous therapy such as isotonic glucose in appropriate quantities. For intravenous use, initial concentrations down to about 0.05 to 0.25 mg/ml of active ingredient are satisfactory.

The active compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.1% of active compound. The percentage of the compositions and preparations may, of course, be varied and may conveniently be between about 2% to about 60% of the weight of the unit. The amount of active compound in such therapeutically useful compositions is such that a suitable dosage will be obtained.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as fatty oil. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed.

The following specific examples illustrate the preparation of the compounds of the present invention.

Example 1

**N-[3-(1H-Imidazol-1-yl)propyl]-5-nitro-2-pyridinamine,(Z)-2-butenedioate (1:1)**

A solution of 8.0 g of 2-chloro-5-nitropyridine, 7.0 g of 1H-imidazole-1-propanamine,13 ml of N,N-diisopropylethylamine, and 200 ml of ethanol is stirred at reflux temperature for 16 hours. The reaction mixture is cooled to room temperature, concentrated in vacuo, treated with 100 ml of saturated sodium bicarbonate, and reconcentrated. The residue is extracted with 200 ml of dichloromethane, dried over magnesium sulfate, filtered and concentrated. The oil is dissolved in 100 ml of acetone, clarified with activated carbon, mixed with 6.0 g of maleic acid in 150 ml of acetone, and cooled to -10°C. The resulting precipitate is collected, washed with acetone, dried in vacuo at 60°, giving 8.0 g of the desired product, mp 134-136°C with decomposition.

Example 2

**N-[3-(1H-Imidazol-1-yl)propyl]-3-nitro-2-pyridinamine**

The title compound is prepared by the procedure of Example 1, using 31.7 g of 2-chloro-3-nitropyridine, 50 g of 1H-imidazole-1-propanamine and 500 ml of ethanol. The desired product is crystallized from 50% ethanol, washed with water, dried at room temperature giving 43.7 g of crude product. A 5.0 g portion is recrystallized from 50% ethanol, washed with 50% ethanol, dried at 60°C giving 3.6 g of pure product, mp 115-116°C.

Example 3

**2-[[3-(1H -Imidazol-1-yl)propyl]amino]-6-methyl-3-pyridinecarbonitrile**

The title compound is prepared by the procedure of Example 1, using 7.6 g of 2-chloro-3-cyano-6-methylpyridine, 12.5 g of 1H-imidazole-1-propanamine and 125 ml of 2-methoxyethanol as the solvent. The residue is crystallized from boiling water giving 3.6 g of the desired product, mp 118-120°C.

Example 4

**2-[[3-(1H -Imidazol-1-yl)propyl]amino]-3-pyridinecarbonitrile**

The title compound is prepared by the procedure of Example 1, using 6.5 g of 2-chloro-3-cyanopyridine, 12.5 g of 1H-imidazole-1-propanamine and 500 ml 2-methoxyethanol as the solvent. The residue is crystallized from ethyl acetate giving 2.7 g of the desired product, mp 90-92°C.

Example 5

**N-[3-(1H -Imidazol-1-yl)propyl]-2-quinolinamine**

A solution of 6.54 g of 2-chloroquinoline, 25 g of phenol, and 0.10 g of potassium iodide is stirred at 100-110°C in an oil bath. Ten grams of 1H-imidazole-1-propanamine is added, the oil bath temperature is increased to 160°C, and the mixture heated for 18 hours. The reaction is cooled to room temperature and treated with 80 ml of 10N NaOH and dichloromethane. The organic layer is separated, washed two times with water, dried over magnesium sulfate and concentrated to an oil. The oil is crystallized from ether, giving 7.7 g of pure product, mp 120-122°C.

Example 6

**N-[3-(1H -Imidazol-1-yl)propyl]-4-methyl-2-quinolinamine**

The title compound is prepared by the procedure of Example 5, using 7.1 g of 2-chlorolepidine, 25 ml of phenol, 0.10 g of potassium iodide and 10 g of 1H-imidazole-1-propanamine. Seven and five tenths grams of the desired product is obtained, mp 117-119°C.

Example 7

6-Chlorophenanthridine

A solution of 19 g of 6(5H)-phenanthridinone, 150 ml of phosphorus oxychloride, and 6 ml of N,N-dimethylaniline is refluxed for 3 hours. The excess phosphorus oxychloride is removed in vacuo, the residue poured into a large volume of ice, and extracted with ether. The organic extract is washed with sodium carbonate, dried over magnesium sulfate and concentrated to an oil. The oil is crystallized from ether, giving 9.3 g of desired product, mp 115-117°C.

Example 8

**N-[3-(1H -Imidazol-1-yl)propyl]-6-phenanthridinamine**

The title compound is prepared by the procedure of Example 5, using 4.27 g of the product from

Example 7, 12 ml of phenol, 0.050 g potassium iodide and 6 ml of 1H-imidazole-1-propanamine. Two and nine tenths grams of the desired product is obtained, mp 149-152°C.

Example 9

**N-[3-(1H-Imidazol-1-yl)propyl]-2-pyridinamine,(E)-2-butenedioate (1:2)**

A solution of 7.9 g of 2-bromopyridine, 13.5 g of 1H-imidazole-1-propanamine, and 25 g of phenol is heated at 165-175°C for 16 hours. The reaction is cooled to room temperature, treated with 50 ml of 10N NaOH and 100 ml of water, and extracted with 500 ml of dichloromethane. The organic layer is dried over sodium sulfate, concentrated in vacuo to an oil, dissolved in 100 ml of acetone and treated with a solution of 12 g fumaric acid in 1200 ml of acetone. After recrystallization from ethanol, 3.9 g of the desired product is obtained, mp 136-138°C.

Example 10

**N-[3-(1H-Imidazol-1-yl)propyl]-6-methyl-2-pyridinamine,(E)-2-butenedioate(2:3)**

The title compound is prepared by the procedure of Example 9, using 5.1 g of 2-chloro-6-methylpyridine, 12.5 g of 1H-imidazole-1-propanamine and 25 g of phenol. Treatment of the residual oil with 7.0 g of fumaric acid gave 3.4 g of the desired fumarate, mp 136-138°C.

Example 11

**N-[3-(1H-imidazol-1-yl)propyl]-5-nitro-2-pyridinamine**

The title compound is prepared by the procedure of Example 1, using 31.7 g of 2-chloro-5-nitropyridine, 51 g of 1H-imidazole-1-propanamine and 500 ml of ethanol. A 5.0 g portion is recrystallized from isopropanol, giving 2.8 g of the desired product, mp 115-116°C.

Example 12

**5-Acetyl-2-[[3-(1H-Imidazol-1-yl)propyl] amino]-6-methyl-3-pyridinecarbonitrile**

A mixture of 5.3 g of 5-acetyl-1,2,3,4-tetrahydro-6-methyl-2-oxo-3-pyridinecarbonitrile prepared as disclosed in U.S. Patent 4,415,580 and 30 ml of phenylphosphonic dichloride is heated at 180°-190°C for 1-1/2 hours. The reaction is poured into 500 ml of ice/water, stirred for 10 minutes, made basic with concentrated ammonium hydroxide, and extracted with dichloromethane. The organic layer is dried over sodium sulfate, passed through hydrous magnesium silicate, and concentrated to give 4.6 g of 2-chloro-3-cyano-5-acetyl-6-methylpyridine. The resulting solid is dissolved in 100 ml of dioxane, treated with 6.5 g of 1H-imidazole-1-propanamine in 100 ml of dioxane, and heated on a steam bath for 16 hours. The reaction mixture is concentrated to dryness in vacuo, the residue treated with 100 ml of water, the insoluble product collected and air dried. The product is dissolved in 150 ml of boiling ethyl acetate, clarified and the filtrate cooled to -10°C. The resulting precipitate is collected, giving 1.6 g of the desired product, mp 160-161°C.

Example 13

1-Bromoisoquinoline

A mixture of 9.0 g of 1-hydroxyisoquinoline and 40 ml of phosphorus tribromide is inserted for 15 minutes into a preheated oil bath at 135°C. The resulting 2 layers are cooled and concentrated in vacuo. The residue is treated with ice, water, and sodium carbonate. The reaction mixture is extracted with chloroform, dried over sodium sulfate and concentrated. The product is purified by column chromatography, yielding 6.8 g of pale yellow crystals, mp 41-43°C.

Example 14

**N-[3-(1H**

**-Imidazol-1-yl)propyl]-1-isoquinolinamine**

A mixture of 3.12 g of 1-bromoisoquinoline and 3.75 g of 1H-imidazole-1-propanamine in 40 ml of phenol is heated with stirring at 100°C for 16 hours. The reaction is cooled, diluted with 75 ml of 10N sodium hydroxide, and extracted with chloroform. The organic layers are combined, dried over sodium sulfate and concentrated in vacuo. The product is recrystallized from ethyl acetate to give 2.5 g of tan crystals, mp 131-132.5°C.

Example 15

3-Dimethylamino-3'-(trifluoromethyl)acrylophenone

A mixture of 250 g of 3-(trifluoromethyl)-acetophenone and 260 ml of N,N-dimethylformamide dimethyl acetal is heated on a steam bath for 18 hours. The reaction is cooled, concentrated in vacuo at 85°C, and shaken with 250 ml of hexane. The crystalline precipitate is collected, triturated with hexane and dried in vacuo at room temperature giving 260 g of product, mp 61-63°C.

Example 16

**1,2-Dihydro-2-oxo-6-(α,α,α-trifluoro-m -tolyl) nicotinonitrile**

A mixture of 8.4 g of 2-cyanoacetamide, 24.3 g of 3-dimethylamino-3'-(trifluoromethyl)acrylophenone, 13.8 g of potassium carbonate and 100 ml of pyridine is refluxed for 18 hours. The reaction is cooled to room temperature, the insolubles collected, slurried with 2x100 ml of water and washed with 100 ml of 10% acetic acid. The solid is suspended in 300 ml of boiling ethanol, 50 ml of N,N-dimethylformamide added to dissolve, clarified and cooled at -10°C. The product is collected, washed with ethanol, and dried at 60°C to yield 7.4 g, mp 260-261°C.

Example 17

2-Chloro-6-[3-(trifluoromethyl)phenyl]-3-pyridinecarbonitrile

Following the procedure of JACS 80, 5481 (1958), 2.9 g of 1,2-dihydro-2-oxo-6-(α,α,α,-trifluoro--m-tolyl) nicotinonitrile is treated with 13 ml of phenyphosphonic dichloride and refluxed for 18 hours. The reaction mixture is cooled, poured into ice water, the pH adjusted to 7 with potassium carbonate and extracted with chloroform. The organic layer is concentrated in vacuo and the residue crystallized from ethanol to give 2.2 g of dark yellow needles, mp 120-121°C.

Example 18

**2-[[3-(1H -Imidazol-1-yl)propyl]amino]-6-[3-(trifluoromethyl)phenyl]-3-pyridinecarbonitrile**

The title compound is prepared by the procedure of Example 9, using 2.1 g of 2-chloro-6-[3-(trifluoromethyl)phenyl-3-pyridinecarbonitrile, 2.0 g of 1H-imidazole-1-propanamine and 10 ml of phenol. The product is purified by chromatography to give 0.76 g of pale yellow crystals.

Example 19

2-Hydroxy-4-methyl-6-methoxyquinoline

To 15 ml of concentrated sulfuric acid heated at 70°C is added 4.14 g of p-acetoacetanisidide in portions so as to maintain the temperature between 79-81°C for 15 minutes. The reaction is poured into 125 ml of ice and allowed to stand at room temperature overnight. The product is collected, washed with water and dried at 50°C to give 0.88 g of off-white crystals, mp 266-270°C.

Example 20

2-Chloro-6-methoxy-4-methylquinoline

A mixture of 10.0 g of 2-hydroxy-4-methyl-6-methoxyquinoline, 8.6 g of phosphorus pentachloride, and 24 ml of phosphorus oxychloride is heated in an oil bath at 125°C for 2-1/2 hours. The reaction is concentrated in vacuo, ice is added slowly and crystals form on standing. The reaction is further diluted with 250 ml of water, filtered, the product collected and air dried to yield 10.25 g of tan crystals, mp 142-144.5°C.

Example 21

**N-[3-(1H-Imidazol-1-yl)propyl]-6-methoxy-4-methyl-2-quinolinamine**

The title compound is prepared by the procedure of Example 9, using 3.15 g of 2-chloro-6-methoxy-4-methylquinoline, 3.75 g of 1H-imidazole-1-propanamine and 40 ml of phenol. The product is purified by chromatography to give 1.18 g of colorless crystals, mp 114-117°C.

Example 22

5,7-Dimethyl-1,8-naphthyridin-2-one

The title compound is prepared by the procedure of A. Mangini and M. Colonna, Gazz. chim. ital 73, 323-29 (1943) using 31.8 g of 7-amino-2,4-dimethyl-1,8-naphthyridine. The product is recrystallized from boiling ethanol to give 10.6 g of crystals, mp 247-249°C.

Example 23

7-Chloro-2,4-dimethyl-1,8-naphthyridine

A mixture of 4.4 g of 5,7-dimethyl-1,8-naphthyridin-2-one and 50 ml of phosphorus oxychloride is refluxed for 30 minutes. The reaction mixture is concentrated in vacuo, treated with ice/water and 10N sodium hydroxide, and extracted with chloroform. The organic layer is dried over sodium sulfate, concentrated in vacuo and recrystallized from acetone to give 3.6 g of mustard colored needles, mp 144-145°C.

Example 24

**N-[3-(1H-Imidazol-1-yl)propyl]-5,7-dimethyl-1,8-naphthyridin-2-amine, (E)-2-butenedioate (1:2)**

The title compound is prepared by the procedure of Example 9, using 3.0 g of 7-chloro-2,4-dimethyl-1,8-naphthyridine, 4 g of 1H-imidazole-1-propanamine and 15 ml of phenol. The resulting oil is treated with 5.4 g of fumaric acid in 550 ml of boiling acetone. The product is recrystallized from ethanol to give 5.3 g of colorless needles.

Example 25

**1,8-Naphthyridin-2(1H)-one**

The title compound is prepared by the procedure of W. Roszkiewicz and M. Wozniak, Synthesis, 1976, 691-2, using 95 g of 2-amino-6-hydroxypyridine sulfate. The product is recrystallized from boiling water to yield 7.9 g of crystals, mp 197-198°C.

Example 26

2-Chloro-1,8-naphthyridine

The title compound is prepared by the procedure of JCS (1967)(C), 1564 using 3.75 g of the product from Example 25 and 75 ml of phosphorus oxychloride. The product is recrystallized from ethanol to give 3.9 g of yellow crystals, mp 139-140°C.

Example 27
**N-[3-(1H -Imidazol-1-yl)propyl]-1,8-naphthyridin-2-amine,(E)-2-butenedioate (1:2)**

The title compound is prepared by the procedure of Example 9, using 33 g of the product from Example 26, 5.0 g of 1H-imidazole-1-propylamine, and 15 ml of phenol. The product is recrystallized from ethanol to give 7.7 g of yellow crystals.

Example 28

1,6-Dihydro-2-methyl-6-oxo-3-pyridinecarbonitrile

The title compound is prepared following the procedure in U.S. Patent #4,657,915 using methyl propiolate and 3-aminocrotononitrile to give 4.2 g of crystals, mp 318-321°C with decomposition.

Example 29

6-Chloro-2-methyl-3-pyridinecarbonitrile

Eight grams of product from Example 28 and 30 ml of phenylphosphonic dichloride is heated with stirring at 180-185°C for 16 hours. The reaction is cooled, poured into 500 ml of ice/water and basified with 200 ml of concentrated ammonium hydroxide. The precipitate is collected, washed with water and air dried. The product is dissolved in dichloromethane, dried over sodium sulfate, passed through hydrous magnesium silicate and concentrated in vacuo to give 6.2 g of a yellow solid, mp 103-105°C.

Example 30

**6-[[3-(1H -Imidazol-1-yl)propyl]amino]-2-methyl-3-pyridinecarbonitrile**

The title compound is prepared by the procedure of Example 1, using 5.7 g of the product from Example 29, 9.4 g of 1H-imidazole-1-propanamine and 25 ml of phenol. The product is recrystallized from diethyl ether to give 3.3 g of yellow crystals, mp 121-123°C.

Example 31

2-Chloro-4,8-dimethylquinoline

A mixture of 20.0 g of 2-hydroxy-4,8-dimethylquinoline, 16.7 g of phosphorus pentoxide, and 50 ml of phosphorus oxychloride is refluxed for 2-1/2 hours. The reaction is cooled, concentrated in vacuo to remove excess phosphorus oxychloride, and the oil poured into ice. The product is collected, washed with water and air dried, mp 61-63°C.

Example 32
**N-[3-(1H -Imidazol-1-yl)propyl]-4,8-dimethyl-2-quinolinamine**

The title compound is prepared by the procedure of Example 9 using 2.87 g of 2-chloro-4,8-dimethylquinoline and 3.75 g of 1H-imidazole-1-propanamine. The product is purified by chromatography and recrystallized from ethyl ether to give 2.7 g of colorless crystals, mp 108-110°C.

Example 33

7-Chloro-5-methyl-1,8-naphthyridin-2-amine

The title compound is prepared by the procedure of Example 23 using 10 g of 7-amino-4-methyl-1,8-naphthyridine-2-one and 50 ml of phosphorus oxychloride to give 2.0 g of yellow crystals.

Example 34
**$N^2$-[3-(1H-Imidazol-1-yl)propyl]-4-methyl-1,8-naphthyridine-2,7-diamine**

The title compound is prepared by the procedure of Example 9 using 3.7 g of Example 33 and 4.8 g of 1H-imidazole-1-propanamine to give 0.37 g of yellow crystals, mp 190-192°C.

Example 35

7-Methyl-1,8-naphthyridin-2-amine

Following the procedure in JOC.30 1608 (1965) 55 g of 2,6-diaminopyridine, 65 g of acetylacetaldehyde dimethyl acetal and 500 ml of phosphoric acid is heated on a steam bath at 90°C for 3 hours. The reaction is poured into 1 kg of ice, neutralized with concentrated ammonium hydroxide and cooled to 0°C. The resulting precipitate is collected, washed with water and dried over phosphorus pentoxide at 60-70°C. The solid is slurried in 400 ml of water, the pH adjusted to PH 10 with 10N sodium hydroxide and the slurry extracted with warm chloroform. The organic layer is dried over sodium sulfate, filtered and concentrated in vacuo. The residue is crystallized from toluene to give 3.27 g of product, mp 180-181°C.

Example 36

7-Methyl 1,8-naphthyridin-2-ol

A mixture of 2.75 g of 7-methyl-1,8-naphthyridin-2-amine and 23 ml of 40% sulfuric acid is cooled to -5°C. One and eight-tenths grams of sodium nitrite is added in portions and the reaction mixture is stirred at -5°C for 1 hour; followed by heating at 80°C for 45 minutes. The reaction is cooled, neutralized with 10N sodium hydroxide and extracted with dichloromethane. The organic layer is dried over sodium sulfate and concentrated to give 2.15 g of product, mp 148-150°C.

Example 37

2-Chloro-7-methyl-1,8-naphthyridine

A mixture of 2.1 g of 7-methyl-1,8-naphthyridin-2-ol and 23 ml of phosphorus oxychloride is refluxed for 1 hour, cooled and concentrated in vacuo. The residue is treated with ice, neutralized with concentrated ammonium hydroxide, and extracted with dichloromethane. The organic layer is concentrated in vacuo to give 2.1 g of product, mp 192-195°C (Lit. mp 215-216°C).

Example 38
**N-[3-(1H-Imidazol-1-yl)propyl]-7-methyl-1,8-naphthyridin-2-amine,(E)-2-butenedioate (1:2)**

The title compound is prepared by the procedure of Example 9 using 2.1 g of 2-chloro-7-methyl-1,8-naphthyridine and 3.0 g 1H-imidazole-1-propanamine. The product is recrystallized from ethanol to give 3.5 g of yellow crystals, mp 173-174°C with decomposition.

Example 39

2-Chloro-5-methyl-1,6-naphthyridine

The title compound is prepared by the procedure of Example 23, using 5-methyl-1,6-naphthyridin-2-(1H)-one and 50 ml phosphorus oxychloride. The product is crystallized from acetone to give 2.8 g of orange crystals, mp 95-97°C.

Example 40
**N-[3-(1H-Imidazol-1-yl)propyl]-5-methyl-1,6-naphthyridin-2-amine,(E)-2-butenedioate (2:3)**

The title compound is prepared by the procedure of Example 9, using 2.4 g of 2-chloro-5-methyl-1,6-naphthyridine and 3.4 g of 1H-imidazole-1-propanamine. The product is recrystallized from ethanol to give 1.67 g of cream colored crystals, mp 178-179°C.

Example 41

2-Benzoylacetanilide

A mixture of 17.3 ml of ethyl benzoylacetate and 9.1 ml of aniline is refluxed for 1 hour. The reaction is allowed to cool and then treated with ethanol and water. The product is collected, recrystallized from ethanol to give 4.86 g of colorless crystals, mp 105-106.5°C.

Example 42

4-Phenylcarbostyril

To 20 ml of concentrated sulfuric acid, 16.4 g of 2-benzoylacetanilide is added in portions at room temperature. The reaction is heated while maintaining the temperature at 105-120°C for 20 minutes. After cooling, the reaction is poured into ice, stirred for 1.5 hours and collected. The product is washed with water and dried to give 9.52 g of cream colored crystals, mp 258-261°C.

Example 43

2-Chloro-4-phenylquinoline

The title compound is prepared by the procedure of Example 23, using 9.52 g of 4-phenylcarbostyril and 40 ml of phosphorus oxychloride. The product is recrystallized from ethanol to give 7.57 g of yellow tan crystals, mp 86.5-88°C.

Example 44

**N-[3-(1H-Imidazol-1-yl)propyl]-4-phenyl-2-quinolinamine**

The title compound is prepared by the procedure of Example 9, using 2.4 g of 2-chloro-4-phenylquinoline and 2.5 g of 1H-imidazole-1-propanamine. The product is recrystallized from ethyl acetate to give 1.13 g of colorless crystals, mp 118-120°C.

Example 45

**6-[[3-(1H-Imidazol-1-yl)propyl]amino]-3-pyridinecarbonitrile**

The title compound is prepared by the procedure of Example 1, using 7.0 g of 2-chloro-5-cyanopyridine and 13.5 g of 1H-imidazole-1-propanamine. The product is recrystallized from toluene to give 4.0 g of crystals, mp 100-102°C.

Example 46

**6-[[3-(1H-Imidazol-1-yl)propyl]amino]-3-pyridinecarboxamide**

The title compound is prepared by the procedure of Example 1, using 6.0 g of 6-chloronicotinamide, 10.5 g of 1H-imidazole-1-propanamine, 75 ml of 2-methoxyethanol and 200 mg of potassium iodide. The product is recrystallized from hot water to give 3.9 g of crystals, mp 143-145°C.

Example 47

**6-Chloro-N**

**-[3-(1H -imidazol-1-yl)propyl]-2-pyridinamine**

A mixture of 1.5 g of 2,6-dichloropyridine and 2.5 g of 1H-imidazole-1-propanamine is heated at 110°C for 2 hours. The solution is added to ice/water, stirred for 30 minutes and collected to give 0.5 g of colorless product, mp 90-93°C.

Example 48

**N-(2-Methylphenyl)-beta-oxo-benzenepropanamide**

A mixture of 34.6 ml of ethyl benzoylacetate and 21.4 g of o-toluidine is refluxed for 15 minutes, cooled and diluted with ethanol/water. The product is recrystallized from ethanol to give 20.1 g of colorless crystals, mp 138-139°C.

Example 49

8-Methyl-4-phenyl-2-quinolinol

The title compound is prepared by the procedure of Example 42, using 20 ml of concentrated sulfuric acid and 17.5 g of N-(2-methylphenyl)-beta-oxo-benzenepropanamide. The product is recrystallized from ethanol to give 2.38 g of colorless crystals, mp 221-222°C.

Example 50

2-Chloro-8-methyl-4-phenylquinoline

The title compound is prepared by the procedure of Example 23, using 40 ml of phosphorus oxychloride and 8.55 g of 8-methyl-4-phenyl-2-quinolinol. The product is recrystallized from ethanol to give 2.86 g of colorless crystals, mp 44-47°C.

Example 51

**N-[3-(1H -Imidazol-1-yl)propyl]-8-methyl-4-phenyl-2-quinolinamine**

The title compound is prepared by the procedure of Example 23, using 2.53 g of 2-chloro-8-methyl-4-phenylquinoline and 2.5 g of 1H-imidazole-1-propanamine. The product is recrystallized from ethyl acetate to give 2.0 g of crystals, mp 85-88°C.

Example 52

**2-[[3-(1H -Imidazol-1-yl)propyl]amino]-6-(4-pyridinyl)-3-pyridinecarbonitrile**

The title compound is prepared by the procedure of Example 1, using 3.5 g of 2-chloro-6-(4-pyridinyl)-3-pyridinecarbonitrile, 4.5 g of 1H-imidazole--1-propanamine and 100 ml dioxane. The product is recrystallized from ethyl acetate to give 1.3 g of crystals, mp 134-135°C.

Example 53

**2-Phenyl-2H -1,2,3-triazolo[4,5-b]pyridin-5-amine**

A mixture of 45.9 g of 3-(phenylazo)-2,6-pyridinediamine, 158.4 g of copper sulfate, 550 ml of pyridine and 330 ml of water is heated at reflux for 4 hours. The reaction is poured into 2.8 L of water, filtered and dried to give 35 g of product.

Example 54

**2-Phenyl-2H -1,2,3,-triazolo[4,5-b]pyridin-5-ol, sodium salt**

To an ice cooled solution of 2.11 g of 2-phenyl-2H-1,2,3-triazolo[4,5-b]pyridin-5-amine and 220 ml of glacial acetic acid is added a solution of 0.70 g of sodium nitrite in 15 ml of concentrated sulfuric acid. The reaction is kept cold for an additional 20 minutes, followed by the addition of a cold solution of 15 ml of concentrated sulfuric acid and 30 ml of water. The resulting solution is heated on a steam bath for 1 hour and allowed to stand at room temperature for 16 hours. The solid is collected, treated with water and excess 10N sodium hydroxide, and recrystallized from water to give yellow crystals.

Example 55

**5-Chloro-2-phenyl-2H -1,2,3-triazolo[4,5-b]-pyridine**

The title compound is prepared by the procedure of Example 23, using 1.0 g of 2-phenyl-2H-1,2,3-triazolo[4,5-b]pyridin-5-ol and 10 ml of phosphorus oxychloride. The product is recrystallized from hexane to give 0.80 g of colorless crystals, mp 151.5-152.5°C.

Example 56

**N-[3-(1H -Imidazol-1-yl)propyl]-2-phenyl-2H -1,2,3-triazolo[4,5-b]pyridin-5-amine**

The title compound is prepared by the procedure of Example 9, using 3.45 g of 5-chloro-2-phenyl-2H-1,2,3-triazolo[4,5-b]pyridine, 3.75 g of 1H-imidazole-1-propanamine, 0.100 g of potassium iodide and 25 ml of phenol. The product is purified by chromatography to give 2.07 g of pale yellow crystals, mp 148-150°C.

Example 57

**6-Chloro-N -[3-(1H -imidazol-1-yl)propyl]-3-nitro-2-pyridinamine, monohydrochloride**

To a cooled solution of 2.1 g of 2,6-di-chloro-3-nitropyridine in 25 ml of acetonitrile is added dropwise 2.55 g of 1H-imidazole-1-propanamine in 25 ml of acetonitrile. The reaction is stirred for 16 hours at room temperature, filtered and the filtrate concentrated in vacuo. The residue is purified by chromatography and treated with ethanolic hydrogen chloride. The product is recrystallized from ethanol to give 1.0 g of crystals, mp 186-187°C.

Example 58

**N-[3-(1H -Imidazol-1-yl)propyl]-N -(5-nitro-2-pyridinyl)acetamide**

To a suspension of 1.28 g of 60% sodium hydride (in oil) in 80 ml of tetrahydrofuran is added 7.41 g of N-[3-(1H-imidazol-1-yl)propyl]-5-nitro-2-pyridinamine (Example 11). The reaction is stirred at room temperature for 10 minutes followed by 15 minutes at reflux temperature. The solution is cooled in ice, 3.12 ml of glacial acetic acid in 20 ml of tetrahydrofuran is added and the reaction is stirred at room temperature for 16 hours. The reaction is concentrated in vacuo, ice water is added and extracted with chloroform. The product is purified by chromatography and recrystallized from toluene to give 2.3 g of crystals, mp 78-80°C.

Example 59

1-(3-Thienyl)ethanone, oxime

A mixture of 54 ml of 3-acetylthiophene, 34.75 g of hydroxylamine hydrochloride, 41 g of sodium

acetate, 150 ml of ethanol and 200 ml of water is refluxed for 6 hours; followed by stirring for 16 hours at room temperature. The reaction is filtered to give 60.3 g of colorless crystals, mp 115-118°C.

Example 60

N-3-Thienylacetamide

To a 0°C solution of 56.4 g of 1-(2-thienyl)-ethanone, oxime in 1.5 liters of diethyl ether is added in portions, while maintaining the temperature at 0°C, 100 g of phosphorus pentachloride. After the addition, the reaction is stirred at 0°C for 2 hours, and at room temperature for 20 hours. The reaction is poured into 1,000 g of ice, neutralized with 10N sodium hydroxide, and extracted with chloroform. Six and three tenths grams of product is purifed by chromatography to give 2.6 g of colorless crystals, mp 148-149°C.

Example 61

5-Chlorothieno[3,2-b]pyridine

The title compound is prepared by the procedure of O. Meth-Cohn,B.Narine, JCS (1981) 1531, using 7.0 g of N-3-thienylacetamide. The product is recrystallized from diethyl ether/hexane to give 2.6 g of yellow crystals, mp 73-74°C.

Example 62

**N-[3-(1H-Imidazol-1-yl)propyl]thieno[3,2-b]-pyridin-5-amine**

The title compound is prepared by the procedure of Example 9, using 2.4 g of 5-chlorothieno[3,2-b]-pyridine and 3.54 g of 1H-imidazole-1-propanamine. The product is purified by chromatography and recrystallized from methanol/diethyl ether to give 0.34 g of yellow crystals, mp 93-94°C.

Example 63

2-(3-Fluorophenyl)-1,4,5,6-tetrahydro-6-oxo-3-pyridinecarbonitrile

To an argon flushed suspension of 13.2 g of 60% sodium hydride (in oil) is added 36.3 g of 3-fluorobenzonitrile and 12.7 ml of dry acetonitrile in 250 ml of dry tetrahydrofuran and the reaction is refluxed for 20 hours. To the refluxing solution, 33 ml of ethyl acrylate in 100 ml of tetrahydrofuran is added over 30 minutes. After refluxing for 4 hours, 125 ml of 3N hydrochloric acid is added dropwise over 30 minutes and the refluxing is continued for an additional 30 minutes. The reaction is concentrated in vacuo, extracted with chloroform and water, and the chloform layer passed through hydrous magnesium silicate. The product is crystallized from chloroform/diethyl ether to give 11.5 g of colorless crystals, mp 200-201°C.

Example 64

1,6-Dihydro-2-(3-fluorophenyl)-6-oxo-3-pyridinecarbonitrile

A mixture of 4.5 g of 2-(3-fluorophenyl)-1,4,5,6-tetrahydro-6-oxo-3-pyridinecarbonitrile, 1.0 g of palladium/carbon, and 15 ml of diphenyl ether is refluxed for 20 hours. The reaction is concentrated in vacuo, chloroform is added, heated to boiling and filtered. The residue is dissolved in chloroform/dimethylformamide, passed through diatomaceous earth and concentrated to give 2.5 g of yellow crystals, mp 276-277°C.

Example 65

6-Chloro-2-(3-fluorophenyl)-3-pyridinecarbonitrile

The title compound is prepared by the procedure of Example 17, using 3.3 g of 1,6-dihydro-2-(3-fluorophenyl)-6-oxo-3-pyridinecarbonitrile and 11 ml of phenyphosphonic dichloride. The product is recrystallized from chloroform/diethyl ether to give 2.5 g of yellow crystals, mp 132-134°C.

Example 66

**2-(3-Fluorophenyl)-6-[[3-(1H -imidazol-1-yl) propyl]amino]-3-pyridinecarbonitrile**

The title compound is prepared by the procedure of Example 9, using 2.2 g of 6-chloro-2-(3-fluorophenyl)-3-pyridinecarbonitrile, 2.4 g of 1H-imidazole-1-propanamine and 10 ml of phenol. The product is purified by chromatography to give 2.1 g of yellow crystals, mp 175-176°C.

Example 67

**3-Amino-3-[3-trifluoromethyl)phenyl]-2-propenenitrile, (Z)**

To a suspension, under argon, of 2.2 g of 60% sodium hydride (washed free of oil with hexane) and 50 ml of diethyl ether is added over 15 minutes a solution of 50 ml diethyl ether, 6.7 ml of 3-(trifluoromethyl)-benzonitrile, 2.9 ml acetonitrile and 0.5 ml t-butanol. The reaction is refluxed for 16 hours, cooled to room temperature and 40 ml of cold water is added dropwise. The organic layer is dried and concentrated to a syrup. The product is crystallized from chloroform/hexane to give 5.6 g of cream crystals, mp 75-77°C.

Example 68

1,4,5,6-Tetrahydro-6-oxo-2-[3-trifluoromethyl) phenyl]-3-pyridinecarbonitrile

To a mixture of 4.2 g of (Z)-3-amino-3-[3-trifluoromethyl)phenyl]-2-propenenitrile in 100 ml of 1,2-dichlorobenzene and 0.80 g of 60% sodium hydride is added dropwise 2.2 ml of ethyl acrylate. The reaction is stirred at room temperature for 10 minutes, refluxed for 18 hours, cooled in ice and added dropwise 10 ml of ice water. The organic layer is concentrated, refluxed with 200 ml of diethyl ether and the product collected as a colorless solid. The solid is crystallized from ethyl acetate to give 0.470 g of colorless rods, mp 181-183°C.

Example 69

1,6-Dihydro-6-oxo-2-[3-(trifluoromethyl)phenyl]-3-pyridinecarbonitrile

The title compound is prepared by the procedure of Example 64, using 10 g of 1,4,5,6-tetrahydro-6-oxo-2-[3-(trifluoromethyl)phenyl]-3-pyridinecarbonitrile, 1.0 g of 10% palladium /carbon and 100 ml diphenyl ether. The product is recrystallized from methanol/diethyl ether to give 1.3 g of colorless crystals, mp 194-196°C.

Example 70

6-Chloro-2-[3-(trifluoromethyl)phenyl]-3-pyridinecarbonitrile

The title compound is prepared by the procedure of Example 17, using 2.5 g of 1,6-dihydro-6-oxo-2--[3-(trifluoromethyl)phenyl]-3-pyridinecarbonitrile and 10 ml of phenylphosphoric dichloride. The product is recrystallized from diethyl ether/hexane to give 1.93 g of yellow crystals, mp 114-115°C.

Example 71

**6-[[3-(1H -Imidazol-1-yl)propyl]amino]-2-[3-(trifluoromethyl)phenyl]-3-pyridinecarbonitrile, (E)-2-butenedioate (1:1)**

The title compound is prepared by the procedure of Example 9, using 1.7 g of 6-chloro-2-[3-(triflurometyl)phenyl]-3-pyridinecarbonitrile, 1.5 g of 1H-imidazole-1-propanamine and 10 ml of phenol. The product is recrystallized from methanol/diethyl ether to give 0.750 g of colorless crystals, mp 129-130°C.

Example 72

1,4,5,6-Tetrahydro-6-Oxo-2-(3,4,5 -trimethoxyphenyl)-3-pyridine-carbonitrile

The title compound is prepared by the procedure of Example 63, using 5.7 g of 60% sodium hydride, 25.2 g of trimethoxybenzonitrile, 250 ml tetrahydrofuran and 5.5 ml of acetonitrile. After the initial 18 hours of reflux, 15 ml of ethyl acrylate is added and the reaction is refluxed for 3 hours. The product is crystallized from methanol/diethyl ether to give 8.8 g of yellow crystals, mp 216-218°C.

Example 73

1,6-Dihydro-6-oxo-2-(3,4,5-trimethoxyphenyl)-3-pyridinecarbonitrile

The title compound is prepared by the procedure of Example 64, using 5.0 g of 1,4,5,6-tetrahydro-6-oxo-2-(3,4,5-trimethoxyphenyl)-3-pyridinecarbonitrile, 1.0 g of 10% palladium/carbon and 15 ml diphenyl ether. The product is recrystallized from chloroform/dimethyl sulfoxide to give 1.0 g of colorless crystals, mp 306-307°C.

Example 74

6-Chloro-2-(3,4,5-trimethoxyphenyl)-3-pyridinecarbonitrile

The title compound is prepared by the procedure of Example 23, using 1.5 g of 1,6-dihydro-6-oxo-2-(3,4,5-trimethoxyphenyl)-3-pyridinecarbonitrile and 50 ml of phosphorus oxychloride. The product is dissolved in chloroform and passed through hydrous magnesium silicate to give 1.7 g of a yellow syrup.

Example 75

**6-[[3-(1H -Imidazol-1-yl)propyl]amino]-2-(3, 4,5-trimethoxyphenyl)-3-pyridinecarbonitrile, (E)-2-butenedioate (1:1)**

The title compound is prepared by the procedure of Example 9, using 1.6 g of Example 74, 13 ml of phenol, 2 crystals of potassium iodide and 3.6 g of 1H-imidazole-1-propanamine. The product is recrystallized from acetone to give 0.430 g of colorless crystals, mp 107-110°C.

Example 76

[(2-Nitrophenyl)methylene]propanedioic acid, diethyl ester

A mixture of 30.0 g of o-nitrobenzaldehyde, 32.0 g of diethylmalonate, 30.0 g of potassium bicarbonate and 72 ml of acetic anhydride is heated at 110°C for 2 hours, cooled and added 3 liters of water. The reaction is extracted with diethyl ether, concentrated in vacuo, dissolved in dichloromethane and passed through a column of silica gel. A 5.0 g sample of product is recrystallized from ethanol to give 3.90 g of yellow crystals, mp 48-51°C.

Example 77

1,2-Dihydro-1-hydroxy-2-oxo-3-quinolinecarboxylic acid, ethyl ester

A solution of 29.3 g of [(2-nitrophenyl)-methylene]propanedioic acid, diethyl ester, 3.0 g of 10% palladium/carbon, and 150 ml of ethyl acetate is reduced catalytically. The reaction is filtered, concentrated in vacuo and crystallized from ethyl acetate to give 15.75 g of product, mp 163-167°C.

Example 78

1,2-Dihydro-1-hydroxy-2-oxo-3-quinolinecarboxamide

Ammonia gas is vigorously bubbled through a mixture of 11.4 g of 1,2-dihydro-1-hydroxy-2-oxo-3-quinolinecarboxylic acid, ethyl ester, 175 ml of methanol, 100 ml of water and 0.20 g of ammonium chloride

until complete solution occurred. The reaction flask is sealed and stirred at room temperature for 5 days. The semisolid reaction is concentrated *in vacuo* and the solid collected to give 9.66 g of product.

Example 79

1-(Acetyloxy)-1,2-dihydro-2-oxo-3-quinolinecarboxamide

A mixture of 8.25 g of 1,2-dihydro-1-hydroxy-2-oxo-3-quinolinecarboxamide and 75 ml of acetic anhydride is heated at 120°C for 0.5 hour. The reaction is filtered hot and concentrated to give 9.9 g of tan crystals, mp 178-193°C.

Example 80

1,2-Dihydro-2-oxo-3-quinolinecarboxamide

A mixture of 6.64 g of 1-(acetyloxy)-1,2-dihydro-2-oxo-3-quinolinecarboxamide, 1.5 g of 10% palladium/carbon, 100 ml glacial acetic acid and 75 ml of ethanol is reduced catalytically. The reaction is warmed on a steam bath, filtered and concentrated *in vacuo* to give 5.10 g of crystalline product, mp 289-293°C.

Example 81

2-Chloro-3-quinolinecarbonitrile

The title compound is prepared by the procedure of Example 23, using 4.6 g of 1,2-dihydro-2-hydroxy-2-oxo-3-quinolinecarboxamide and 50 ml of phosphorus oxychloride. The product is crystallized from water to give 3.85g of tan crystals, mp 165-166°C.

Example 82

**2-[[3-(1H-Imidazol-1-yl)propyl]amino]-3-quinolinecarbonitrile**

The title compound is prepared by the procedure of Example 9, using 1.9 g of 2-chloro-3-quinolinecarbonitrile, 0.10 g of potassium iodide, 25 ml of phenol and 2.5 g of 1H-imidazole-1-propanamine. The product is purified by chromatography and recrystallized from acetone to give 0.32 g of yellow crystals, mp 97-99°C.

Example 83

**N-[3-(1H-Imidazol-1-yl)propyl]-N-(4-methyl-2-quinolinyl)acetamide,monoacetate**

To a stirred solution of 2.66 g of N-[3-(1H-imidazol-1-yl)propyl]-4-methyl-2-quinolinamine (Example 6), in 20 ml of dry pyridine containing 0.10 g of 4-dimethylamino-pyridine is added dropwise over 10 minutes, 3 ml of acetic anhydride. The reaction is stirred at room temperature for 5 days, concentrated *in vacuo* and purified by chromatography. The product is triturated with acetone to give 2.43 g of an orange gum.

Example 84

**2-[[3-(1H-Imidazol-1-yl)-2-methylpropyl]amino] 6-methyl-3-pyridinecarbonitrile, dihydrochloride**

The title compound is prepared by the procedure of Example 1, using 5.0 g of 2-chloro-6-methyl-3-pyridinecarbonitrile, 4.7 g of β-methyl-1H-imidazole-1-propanamine and 100 ml of 2-methoxyethanol. The product is recrystallized from ethanol/ether to give 3.0 g of tan crystals, mp 211-213°C.

Example 85

**2-[[3-(1H-Imidazol-1-yl)butyl]amino]-6-methyl-3-pyridinecarbonitrile, dihydrochloride**

The title compound is prepared by the procedure of Example 1, using 3.5 g of β-methyl-1H-imidazole-1-propanamine, 2.5 g of 2-chloro-6-methyl-3-pyridinecarbonitrile, 5 g of sodium carbonate and 100 ml of 2-methoxyethanol. The product is recrystallized from ethanol/ether to give 0.74 g of crystals, mp 193-196°C.

Example 86

**N-[3-(1H-Imidazol-1-yl)-2-methylpropyl]-6-phenanthridinamine**

The title compound is prepared by the procedure of Example 9, using 4.3 g of 6-chlorophenanthridine, 12 ml of phenol, a catalytic amount of potassium iodide, 2.8 g of β-methyl-1H-imidazole-1-propanamine and 2.0 g of sodium bicarbonate. The product is crystallized from diethyl ether to give 1.6 g of crystals, mp 116-117°C.

Example 87

**N-[3-(1H-Imidazol-1-yl)butyl]-4-methyl-2-quinolinamine**

The title compound is prepared by the procedure of Example 9, using 3.55 g of 2-chlorolepidine, 0.10 g of potassium iodide, 20 ml of phenol and 5.56 g of β-methyl-1H-imidazole-1-propanamine. The product is crystallized from diethyl ether to give 3.78 g of colorless crystals, mp 145-147°C.

Example 88

**N-[3-(1H-Imidazol-1-yl)-2-methylpropyl]-4-methyl-2-quinolinamine**

The title compound is prepared by the procedure of Example 9, using 3.55 g of 2-chlorolepidine, 0.10 g of potassium iodide, 20 ml of phenol and 5.56 g of β-methyl-1H-imidazole-1-propanamine. The product is recrystallized from ethyl acetate to give 2.73 g of colorless crystals, mp 142-143°C.

Example 89

**N-[3-(1H-Imidazol-1-yl)butyl]-6-phenanthridinamine**

The title compound is prepared by the procedure of Example 9, using 4.3 g of 6-chlorophenanthridine, 2.8 g of β-methyl-1H-imidazole-1-propanamine, 14 ml of phenol and 2 g of sodium bicarbonate. The product is recrystallized from chloroform to give 2.1 g of crystals, mp 141-142°C.

Example 90

**2-[[5-(1H-Imidazol-1-yl)pentyl]amino]-6-methyl-3-pyridinecarbonitrile**

The title compound is prepared by the procedure of Example 1, using 2.5 g of 2-chloro-6-methyl-3-pyridinecarbonitrile, 7.6 g of 1H-imidazole-1-pentanamine and 100 ml of 2-methoxy-ethanol. The product is recrystallized from diethyl ether to give 3.2 g of crystals, mp 68-70°C.

Example 91

6-Methyl-2-[(3-pyridinylmethyl)amino]-3-pyridinecarbonitrile

The title compound is prepared by the procedure of Example 9, using 4.4 g of 3-pyridinemethanamine, 3.1 g of 2-chloro-6-methyl-3-pyridinecarbonitrile, 0.10 g of potassium iodide and 15 ml of phenol. The product is recrystallized from diethyl ether/hexane to give 1.5 g of crystals, mp 112-115°C.

Example 92

6-Methyl-2-[(2-pyridinylmethyl)amino]-3-pyridinecarbonitrile

The title compound is prepared by the procedure of Example 1, using 8.6 g of 2-pyridinemethanamine, 6.2 g of 2-chloro-6-methyl-3-pyridinecarbonitrile and 200 ml of ethanol. The product is recrystallized from diethyl ether to give 4.7 g of colorless crystals, mp 70-71°C.

Example 93

6-Methyl-2-[(4-pyridinylmethyl)amino]-3-pyridinecarbonitrile

The title compound is prepared by the procedure of Example 1, using 8.6 g of 4-pyridinemethanamine, 6.2 g of 2-chloro-6-methyl-3-pyridinecarbonitrile and 200 ml of ethanol. The product is recrystallized from diethyl ether to give 0.91 g of colorless crystals, mp 137-139°C.

Example 94

2-[(2-Pyridinylmethyl)amino]-3-pyridinecarbonitrile

The title compound is prepared by the procedure of Example 1, using 8.6 g of 2-pyridinemethanamine and 5.5 g of 2-chloro-3-pyridinecarbonitrile. The product is crystallized from chloroform to give 5.4 g of crystals, mp 80-82°C.

Example 95

**4-Methyl-N**
**-[2-(3-pyridinyl)ethyl]-2-quinolinamine,dihydrochloride**

The title compound is prepared by the procedure of Example 9, using 3.55 g of 2-chlorolepidine, 20 ml of phenol, 0.10 g of potassium iodide and 4.88 g of 3-pyridineethanamine. The product is crystallized from ethanolic hydrogen chloride to give 3.52 g of crystals, mp 260-268°C.

Example 96

6-Methyl-2-[[2-(2-pyridinyl)ethyl]amino]-6-methyl-3-pyridinecarbonitrile

The title compound is prepared by the procedure of Example 9, using 3.1 g of 2-chloro-6-methyl-3-pyridinecarbonitrile, 4.9 g of 2-pyridineethanamine, 0.10 g of potassium iodide and 15 ml of phenol. The product is recrystallized from diethyl ether to give 0.87 g of gold crystals, mp 104-105°C.

Example 97

6-Methyl-2-[[2-(4-pyridinyl)ethyl]amino]-3-pyridinecarbonitrile, dihydrochloride

The title compound is prepared by the procedure of Example 9, using 3.1 g of 2-chloro-6-methyl-3-pyridinecarbonitrile, 4.9 g of 4-pyridineethanamine 0.10 g of potassium iodide and 15 ml of phenol. The product is crystallized from ethanol to give 2.6 g of tan crystals, mp 215-218°C with decomposition.

Example 98

**4-Methyl-N**
**-[3-(3-pyridinyl)propyl]-2-quinolinamine, dihydrochloride**

The title compound is prepared by the procedure of Example 9, using 6.8 g of 3-pyridinepropanamine, 4.4 g of 2-chloro-4-methylquinoline, 25 g of phenol and 0.20 g of potassium iodide. The free base is recrystallized from dichloromethane/hexane, mp 111-112°C. The dihydrochloride is recrystallized from ethanol/acetone, mp 210-212°C.

Example 99

6-Methyl-2-[[3-(3-pyridinyl)propyl]amino] 3-pyridinecarbonitrile, dihydrochloride

The title compound is prepared by the procedure of Example 9, using 3.1 g of 2-chloro-6-methyl-3-pyridinecarbonitrile, 5.4 g of 3-pyridinepropanamine, 0.10 g potassium iodide and 15 ml of phenol. The product is recrystallized from methanol/diethyl ether to give 2.2 g of tan crystals, mp 188-190°C.

Example 100
**N-(3-Nitro-2-pyridinyl)-4-pyridinebutanamine, (E)-2-butenedioate (1:1)**

The title compound is prepared by the procedure of Example 1, using 5.4 g of 2-chloro-3-nitropyridine, 5.0 g of 4-pyridinebutanamine, 10 ml of N,N-diisopropylethylamine and 100 ml of dioxane. The free base is recrystallized from hexane to give 0.7 g of yellow crystals, mp 44°-46°C. The fumarate is crystallized from acetone, mp 126-127°C.

Example 101
**N-[4-(1H**
**-Imidazol-1-yl)butyl]-4-methyl-2-quinolinamine**

The title compound is prepared by the procedure of Example 9, using 5.33 g of 2-chlorolepidine, 8.35 g of 1H-imidazole-1-butanamine, 0.10 g of potassium iodide and 25 ml of phenol. The product is purified by chromatography and recrystallized from diethyl ether to give 5.0 g of colorless crystals, mp 109-111°C.

Example 102
**N-[4-(3-pyridinyl)butyl]-6-phenanthridinamine**

The title compound is prepared by the procedure of Example 9, using 4.3 g of 6-chlorophenanthridine, 3.0 g of 3-pyridinebutanamine, 12 ml of phenol and 2.0 g of sodium bicarbonate. The free base is purified by chromatography and the dihydrochloride recrystallized from ethanol/diethyl ether to give 4.4 g of colorless crystals, mp 121-124°C.

Example 103

**4-Methyl-N**
**-4-(3-pyridinylbutyl)-2-quinolinamine, dihydrochloride**

The title compound is prepared by the procedure of Example 9, using 2-chlorolepidine, 3-pyridinebutanamine, 20 ml of phenol and 0.10 g of potassium iodide. The free base is purified by chromatography and the dihydrochloride recrystallized from ethanol/ethyl acetate to give 4.64 g of colorless crystals, mp 211-213°C.

Example 104
**N-[4-(1H**
**-Imidazol-1-yl)butyl]-6-phenanthridinamine**

The title compound is prepared by the procedure of Example 9, using 4.3 g of 6-chlorophenanthridine, 4.2 g of 1H-imidazole-1-butanamine, 4.0 g of sodium bicarbonate and 13 ml of phenol. The product is purified by chromatography and recrystallized from acetone to give 3.2 g of colorless crystals, mp 66-68°C.

Example 105
**N-[4-(3-Pyridinyl)butyl]-1-isoquinolinamine, dihydrochloride**

The title compound is prepared by the procedure of Example 9, using 3.12 g of 1-bromoisoquinoline, prepared as in Example 13 4.5 g of 3-pyridinebutanamine and 20 ml of phenol. The product is recrystallized from ethanol to give 2.33 g of tan crystals, mp 208-211°C with decomposition.

Example 106

**6-Methoxy-4-methyl-N**

**-[4-(3-pyridinyl)butyl]-2-quinolinamine**

The title compound is prepared by the procedure of Example 9, using 3.15 g of 2-chloro-6-methoxy-4-methylquinoline, 4.50 g of 3-pyridinebutanamine, 0.10 g of potassium iodide and 20 ml of phenol. The product is recrystallized from ethanol to give 2.72 g crystals, mp 108-110°C.

Example 107

**4,8-Dimethyl-N**
**-[4-(3-pyridinyl)butyl]-2-quinolinamine**

The title compound is prepared by the procedure of Example 9, using 2.87 g of 2-chloro-4,8-dimethylquinoline, 4.50 g of 3-pyridinebutanamine, 0.10 g potassium iodide and 25 ml of phenol. The product is recrystallized from ethanol to give 2.36 g of colorless crystals, mp 88-89.5°C.

Example 108

6-Methyl-2-[[4-(3-pyridinyl)butyl]amino]-3-pyridinecarbonitrile

The title compound is prepared by the procedure of Example 9, using 3.0 g of 2-chloro-6-methyl-3-pyridinecarbonitrile, 6.0 g of 3-pyridinebutanamine and 20 ml of phenol. The product is recrystallized from diethyl ether/hexane to give 1.1 g of colorless crystals, mp 53-55°C.

Example 109

**5,7-Dimethyl-N**
**-[4-(3-pyridinyl)butyl]-1,8-naphthyridin-2-amine,(E)-2-butenedioate (1:2)**

The title compound is prepared by the procedure of Example 9, using 1.3 g of 7-chloro-2,4-dimethyl-1,8-naphthyridine, 2.0 g of 3-pyridinebutanamine and 20 ml of phenol. The product is recrystallized from ethanol to give 0.80 g of yellow crystals, mp 123-125°C.

Example 110

**4-Phenyl-N**
**-[4-(3-pyridinyl)butyl]-2-quinolinamine**

The title compound is prepared by the procedure of Example 9, using 2.4 g of 2-chloro-4-phenylquinoline, 3.0 g of 3-pyridinebutanamine, 0.10 g of potassium iodide and 25 ml of phenol. The product is recrystallized from ethanol to give 1.14 g of colorless crystals, mp 100-203°C.

Example 111

**8-Methyl-4-phenyl-N**
**-[4-(3-pyridinyl)butyl]-2-quinolinamine**

The title compound is prepared by the procedure of Example 9, using 2.53 g of 2-chloro-8-methyl-4-phenylquinoline, 3.0 g of 3-pyridinebutanamine, 0.10 g potassium iodide and 25 ml of phenol. The product is purified by chromatography and recrystallized from diethyl ether to give 1.40 g of tan crystals, mp 95-98°C.

Example 112

6-Methyl-2-[[4-(4-pyridinyl)butyl]amino]-3-pyridinecarbonitrile, dihydrochloride

The title compound is prepared by the procedure of Example 9, using 3.1 g of 2-chloro-6-methyl-3-pyridinecarbontrile, 6.0 g of 4-pyridinebutanamine, 0.10 g potassium iodide and 15 ml of phenol. The product is recrystallized from ethanol to give 3.1 g of colorless crystals, mp 93-94°C.

Example 113

6-Methyl-2-[[3-(4-pyridinyl)butyl]amino]-3-pyridinecarbonitrile, dihydrochloride

The title compound is prepared by the procedure of Example 9, using 2.3 g of 2-chloro-6-methyl-3-pyridinecarbonitrile, 4.5 g of b-methyl-3-(4-pyridine)propanamine, 0.10 g of potassium iodide and 10 ml of phenol. The product is recrystallized from ethanol to give 2.4 g of colorless crystals, mp 90-92°C.

Example 114

**N-[3-(1H -imidazol-1-yl)propyl]-7-(trifluoromethyl)-4-quinolinamine**

The title compound is prepared by the procedure of Example 9, using 11.6 g of 4-chloro-7-trifluoromethylquinoline, 15 g of 1H-imidazole-1-propanamine, 30 g of phenol and 0.10 g of potassium iodide. The product is recrystallized from diethyl ether to give 6.9 g of crystals, mp 162-161°C.

Example 115

**7-Chloro-N -[3-(1H -imidazol-1-yl)propyl]-4-quinolinamine**

The title compound is prepared by the procedure of Example 9, using 10 g of 4,7-dichloroquinoline, 15 g of 1H-imidazole-1-propanamine and 30 g of phenol. The product is recrystallized from acetone/water to give 1.8 g of crystals, mp 180-182°C.

Example 116

**N-[3-(1H -imidazol-1-yl)propyl]-9-acridinamine**

The title compound is prepared by the procedure of Example 9, using 4.26 g of 9-chloroacridine, 6 ml of 1H-imidazole-1-propanamine, 0.05 g of potassium iodide and 12 g of phenol. The product is recrystallized from ethanol to give 2.2 g of crystals, mp 172-175°C.

Example 117

4-Hydroxy-3-quinolinecarbonitrile

A mixture of 15.3 g of 4-nitroquinoline N-oxide, 260 ml of N,N-dimethylformamide and 10.5 g of potassium cyanide is heated in an oil bath for 5 hours at 80-90°C. The reaction is concentrated in vacuo, treated with water and chloroform, filtered through diatomaceous earth, and the layers separated. The water layer is cooled, made weakly acidic (pH 4-5), and allowed to stand overnight. The product is collected to give 5.85 g of tan crystals, mp 268-271°C.

Example 118

4-Chloro-3-quinolinecarbonitrile

The title compound is prepared by the procedure of Example 9, using 1.6 g of 4-hydroxy-3-quinolinecarbonitrile and 50 ml of phosphorus oxychloride. The product is recrystallized from hexane to give 0.78 g of yellow crystals, mp 121-123°C. (Lit mp 129-130°C)

Example 119

**4-[[3-(1H -imidazol-1-yl)propyl]amino]-3-quinolinecarbonitrile**

The title compound is prepared by the procedure of Example 1, using 2.85 g of 4-chloro-3-quinolinecarbonitrile, 4.0 g of 1H-imidazole-1-propanamine and 100 ml acetonitrile. The product is recrystallized from acetonitrile to give 2.86 g of crystals, mp 184-186°C.

## Claims

1. A compound selected from the group consisting of those of the formula:

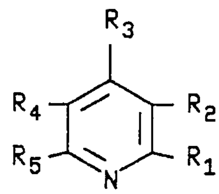

wherein $R_1$ is selected from the group consisting of hydrogen, halogen, or

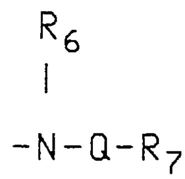 ;

$R_2$ is selected from the group consisting of hydrogen, nitro, nitrile or $R_1$ and $R_2$ taken together can be -CH=CH-CH=CH-; $R_3$ is selected from the group consisting of hydrogen, alkyl $C_1$-$C_3$, phenyl,

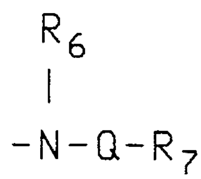

or $R_2$ and $R_3$ taken together can be -CH=CH-CH=CH-; $R_4$ is selected from the group consisting of hydrogen, nitro, nitrile, alkyl $C_1$-$C_3$,

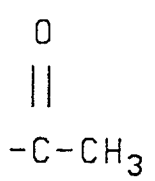, or 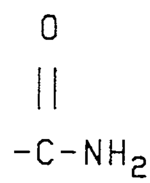 ;

or $R_3$ and $R_4$ taken together is:

-CH=CH-CH=CH- ;

$R_5$ is selected from the group consisting of hydrogen, alkyl $C_1$-$C_3$, halogen, phenyl, 3-(trifluoromethyl)-phenyl, 3-fluorophenyl, 3,4,5-trimethoxyphenyl, and 2,3 or 4-pyridyl or $R_4$ and $R_5$ taken together is selected from the following formulae:

$-CH=CH-CH=CH-$ ,

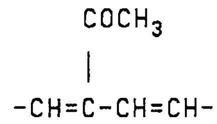 , 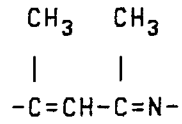 ,

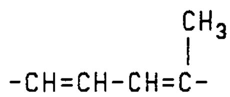 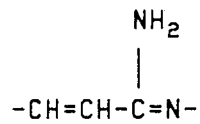 ,

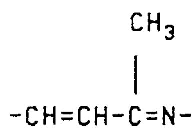 , 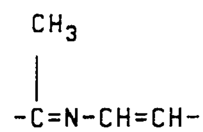 ,

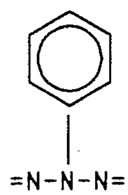 ,

$-S-CH=CH-$ ;

$R_6$ is selected from either hydrogen or acetyl; $R_7$ is selected from 2,3 or 4-pyridyl or 1-imidazolyl; Q is selected from the group consisting of $-(CH_2)_n$ straight or branched chain alkyl where n is an integer from 1 to 5; with the proviso that, in all instances, either $R_1$ or $R_3$ must be:

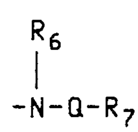

as hereinbefore defined; and the pharmacologically acceptable salts thereof.

**2.** A compound selected from the group consisting of those of the formula:

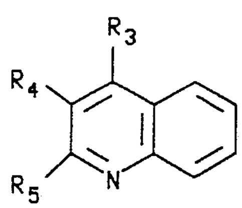 or 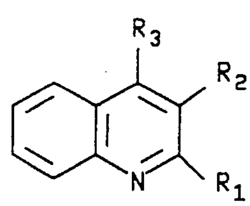

wherein $R_1$ is selected from the group consisting of hydrogen, halogen, or

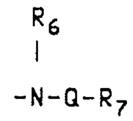 ;

$R_2$ is selected from the group consisting of hydrogen, nitro, or nitrile; $R_3$ is selected from the group consisting of hydrogen, alkyl $C_1$-$C_3$, phenyl, or

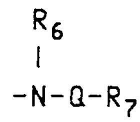

$R_4$ is selected from the group consisting of hydrogen, nitro, nitrile, alkyl $C_1$-$C_3$,

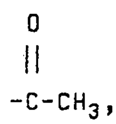, or 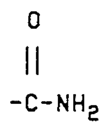 ;

$R_5$ is selected from the group consisting of hydrogen, alkyl $C_1$-$C_3$, halogen, phenyl, 3-(trifluoromethyl)-phenyl, 3-fluorophenyl, 3,4,5-trimethoxyphenyl, and 2,3 or 4-pyridyl; $R_6$ is selected from either hydrogen or acetyl; $R_7$ is selected from 2,3 or 4-pyridyl or 1-imidazolyl; Q is selected from the group consisting of $-(CH_2)_n$ straight or branched chain alkyl where n is an integer from 1 to 5; with the proviso that, in all instances, either $R_1$ or $R_3$ must be:

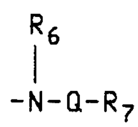

as hereinbefore defined; and the pharmacologically acceptable salts thereof.

3. A compound selected from the group consisting of those of the formulae:

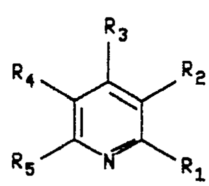

wherein $R_1$ is selected from the group consisting of hydrogen, halogen or

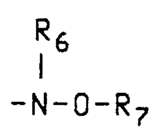 ;

$R_2$ is selected from the group consisting of hydrogen, nitro or nitrile; $R_3$ is selected from the group consisting of hydrogen, alkyl $C_1$-$C_3$, phenyl or

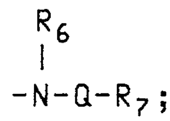

and $R_4$ and $R_5$ taken together is selected from the following formulae:

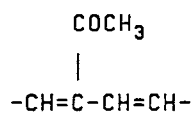 , 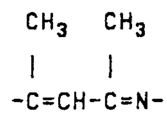 ,

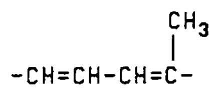 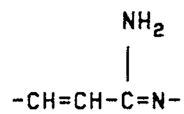 ,

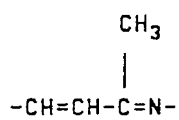 , 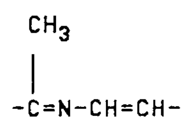 ,

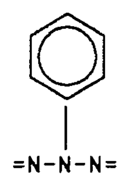 ,

-S-CH=CH- ;

$R_6$ is selected from either hydrogen or acetyl; $R_7$ is selected from either 2,3, or 4-pyridyl or 1-imidazolyl; Q is selected from the group consisting of $-(CH_2)_n$, where n is an integer from 1 to 5, straight or branched chain alkyl; with the proviso that in all instances, either $R_1$ or $R_3$ must be the substituent:

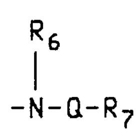

as hereinbefore defined; and the pharmacologically acceptable salts thereof.

**4.** The compound according to Claim 1; N-[3-(1H-Imidazol-1-yl)propyl]-2-quinolinamine.

**5.** A method of inhibiting thromboxane synthetase enzyme in a mammal which comprises administering internally to said mammal a thromboxane synthetase enzyme inhibiting amount of a compound of

Claim 1.

6. A thromboxane synthetase enzyme inhibiting composition of matter in dosage unit form comprising from about 10 mg to about 700 mg of a compound of Claim 1 in association with a pharmacologically acceptable carrier.

7. The method of inhibiting hypertension in a mammal which comprises administering to said mammal a hypotensive amount of a compound of Claim 1.

8. The method of inhibiting arrhythmia in a mammal which comprises administering internally to said mammal an arrhythmia inhibiting amount of a compound of Claim 1.

9. A process for producing a compound of the formula:

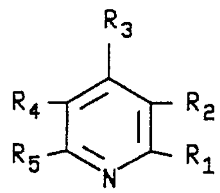

wherein $R_1$ is selected from the group consisting of hydrogen, halogen, or

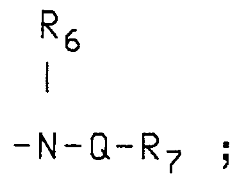

$R_2$ is selected from the group consisting of hydrogen, nitro, nitrile or $R_1$ and $R_2$ taken together can be -CH=CH-CH=CH-; $R_3$ is selected from the group consisting of hydrogen, alkyl $C_1$-$C_3$, phenyl,

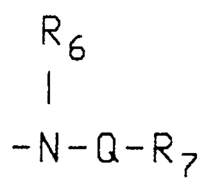

or $R_2$ and $R_3$ taken together can be -CH=CH-CH=CH-; $R_4$ is selected from the group consisting of hydrogen, nitro, nitrile, alkyl $C_1$-$C_3$,

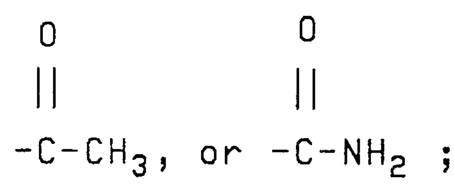

or $R_3$ and $R_4$ taken together is:

-CH=CH-CH=CH- ;

$R_5$ is selected from the group consisting of hydrogen, alkyl $C_1$-$C_3$, halogen, phenyl, 3-trifluorophenyl, 3-fluorophenyl, 3,4,5-trimethoxyphenyl, and 2,3 or 4-pyridyl or $R_4$ and $R_5$ taken together is selected from

the following formulae:

$-CH=CH-CH=CH-$ ,

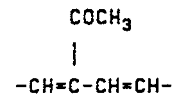 , 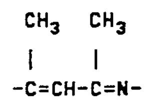 ,

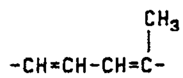 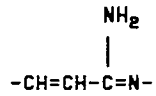 ,

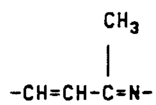 , 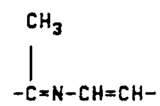 ,

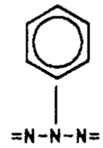 ,

$-S-CH=CH-$ ;

$R_6$ is selected from either hydrogen or acetyl; $R_7$ is selected from 2,3 or 4-pyridyl or 1-imidazolyl; Q is selected from the group consisting of $-(CH_2)_n$, straight or branched chain alkyl where n is an integer from 1 to 5; with the proviso that, in all instances, either $R_1$ or $R_3$ must be:

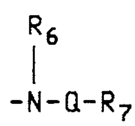

as hereinbefore defined; and the pharmacologically acceptable salts thereof; which comprises, reacting a substituted halo nitrogen-containing ring system of the formula:

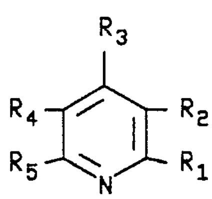

with an approxpriate $R_7$-Q-amine in an appropriate solvent at reflux temperature.

**10.** A process for producing a compound of the formula:

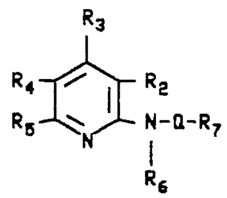

or

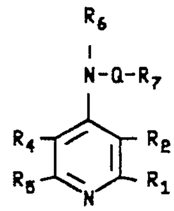

wherein $R_2$-$R_7$ and Q are as defined in claim 27, which comprises reacting a novel substituted nitrogen containing ring system of the formulae:

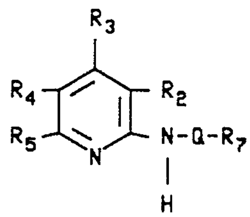

or

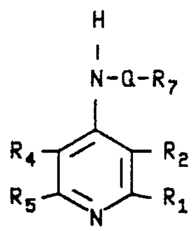

with sodium hydride in tetrahydofuran, adding an appropriate anhydride and heating at reflux temperature for 15 minutes to 6 hours.